# EUROPEAN PATENT APPLICATION

(11) **EP 4 001 299 A1**
(43) Date of publication of application: **25.05.2022**
(21) Application number: 20840533.2
(22) Date of filing: 17.07.2020
(51) Int. Cl.: C07K 14/195, C12N 5/071, C12N 15/31, C12N 11/02

(54) **ADHESIVE PROTEIN**

(30) Priority: 18.07.2019 JP 2019132488
(71) Applicant: National University Corporation Tokai National Higher Education and Research System, Nagoya-shi, Aichi 464-8601 (JP)
(72) Inventor: HORI, Katsutoshi, Nagoya-shi, Aichi 464-8601 (JP)
(74) Representative: Engelhard, Markus
(86) International application number: PCT/JP2020/027921
(87) International publication number: WO 2021/010481

(57) **Abstract**

The present invention provides a novel polypeptide. The polypeptide found by the inventors of the present invention does not have self-cohesive properties and has adhesive properties. The adhesive polypeptide according to the present invention comprises a fragment of the amino acid sequence represented by SEQ ID NO: 1. The adhesive polypeptide according to a specific embodiment of the present invention includes, from among the amino acid sequence represented by SEQ ID NO: 1, amino acids at positions 22-50 and positions 161-175, and also includes, from among amino acids at positions 2847-3093, deletion of a region required to impart self-cohesive properties.

## Description

### [Technical Field]

The present disclosure relates to an adhesive polypeptide without a self-aggregation property and use thereof.

### [Background Art]

*Acinetobacter* sp. Tol5 (genus *Acinetobacter* bacteria Tol5 strain) previously isolated from a biofilter by the inventor are non-pathogenic gram-negative bacteria that have a high cell self-aggregation property and exhibits high adhesive properties to various material surfaces, from various hydrophobic plastic carriers to hydrophilic glass and even metal surfaces. A novel bacterial nanofiber present on the surface layer of a bacterial cell as a factor which brings about such adhesive properties that have not been reported in other microorganisms has been discovered, and a new protein constituting the nanofiber has also been identified. This protein belongs to the trimeric autotransporter adhesin (TAA) family of Gram-negative bacteria and is named AtaA by the inventors (Non-Patent Literature 1). Proteins of the TAA family form a homotrimer and has a common basic structure of signal peptide-head-neck-stalk-membrane anchor from the amino terminus to the carboxy terminus (Non Patent Literature 2). A membrane anchor domain is also referred to as a translocator domain, which forms a beta barrel on the outer membrane and has a function of extracellularly transporting, and presenting to the cell surface layer as a fiber, a passenger domain consisting of head-neck-stalk after a signal peptide is cleaved at the periplasmic space. The head side of a passenger domain is the tip of fiber, corresponding to the amino terminus side of a mature protein. However, there are TAAs that are small with about 300 amino acid residues in a constituent monomer polypeptide chain and those that are large with more than 3000 amino acid residues. The type and arrangement of domains and motifs constituting an amino acid sequence, especially a passenger domain, are diverse. A monomer polypeptide chain that constitutes AtaA discovered by the inventor consists of 3630 amino acids (length including the signal peptide), and is one of the largest among TAAs. This has a unique primary structure with a plurality of long repeat sequences arranged in a mosaic pattern on a long stalk. The repeat sequence is formed by many types of domains repeating and coexisting. The head is also one of the domains. There is one more in the stalk towards the membrane anchor, aside from the tip of the fiber.

AtaA is extremely useful for immobilization of microorganisms and is expected to be applied to various bioprocesses (see, for example, Patent Literatures 1 and 2). Furthermore, in a previous patent application (Patent Literature 3), the inventors reported that various functional peptides and proteins can be introduced into AtaA and presented on the surface of a microorganism. Note that, in Patent Literature 1, AtaA and the gene encoding it (ataA gene) were referred to as AadA and aadA genes, respectively.

### [Citation List]

### [Patent Literature]

[PTL 1] WO 2009/104281
[PTL 2] WO 2014/156736
[PTL 3] WO 2015/030171

### [Non Patent Literature]

[NPL 1] Ishikawa, M.; Nakatani, H.; Hori, K., AtaA, a new member of the trimeric autotransporter adhesins from Acinetobacter sp. Tol 5 mediating high adhesiveness to various abiotic surfaces. PLoS One 2012,7,(11),e48830.
[NPL 2] Linke, D.; Riess, T.; Autenrieth, I.B.; Lupas, A.; Kempf, V.A., Trimeric autotransporter adhesins: variable structure, common function. Trends Microbiol.2006, 14, (6), 264-270.

### [Summary of Invention]

### [Solution to Problem]

The inventors, as a result of diligent studies, discovered that an AtaA protein can exert an adhesive property and self-aggregation property even when only a passenger domain is detached from the cell surface layer and purified. It was also discovered that the same domain is responsible for an adhesive property and self-aggregation property. Despite of the above, an unexpected new fact was found, i.e., an AtaA fragment that lacks a self-aggregation property while maintaining an adhesive property can be obtained by deleting a domain that is different from a domain responsible for self-aggregation from an AtaA passenger domain detached from cells. The present disclosure also relates to applications of the adhesive polypeptide of the present disclosure, e.g., immobilization and/or adhesion method of a functionality-imparting entity and the like.

The improved AtaA in the present disclosure is not produced from a cell, but is found in a protein from which AtaA is separated and purified. In one specific embodiment, one feature can be a lack of a C-terminal membrane binding site for this purpose. Absence of a signal peptide as an adhesive protein can also be one non-limiting representative feature. These features can be utilized as a protein material detached from microorganisms unlike conventionally known AtaA. It was elucidated in the present disclosure that the improved AtaA self-aggregates without Chead, when present on a cell. However, an AtaA fragment loses a self-aggregation property when detached from a cell and Chead is deleted. These features are not a feature of conventional AtaA.

Therefore, the present disclosure provides the following.
(1) A polypeptide comprising a fragment of the amino acid sequence set forth in SEQ ID NO: 1 or a variant sequence thereof, wherein the polypeptide does not have a self-aggregation property and has an adhesive property.
(2)The polypeptide according to any of the preceding items, comprising a sequence corresponding to amino acid positions 22 to 50 and 161 to 175 of the amino acid sequence set forth in SEQ ID NO: 1 or a variant sequence thereof, and one or more amino acid substitutions, additions, deletions, or combinations thereof in a range of a sequence corresponding to amino acid positions 2847 to 3093 of the amino acid sequence set forth in SEQ ID NO: 1.
(3)The polypeptide according to any of the preceding items, wherein the range of a sequence corresponding to amino acid positions 2847 to 3093 of the amino acid sequence set forth in SEQ ID NO: 1 is a range corresponding to amino acid positions 2855 to 3093 of the amino acid sequence set forth in SEQ ID NO: 1.
(4) The polypeptide according to any of the preceding items wherein the entire sequence corresponding to amino acid positions 2855 to 3093 of the amino acid sequence set forth in SEQ ID NO: 1 is deleted.
(5) A polypeptide, which is
   (A) a polypeptide comprising, in a sequence corresponding to amino acid positions 1 to 3518 of the amino acid sequence set forth in SEQ ID NO: 1 or a variant sequence thereof or a fragment thereof,
      at least one or more amino acid substitutions, additions, deletions, or combinations thereof in a sequence corresponding to amino acid positions 2855 to 3093 of SEQ ID NO: 1, and
      a sequence having a sequence corresponding to amino acid positions 22 to 50 and 161 to 175 of SEQ ID NO: 1 or a variant sequence thereof,
   (B) a polypeptide comprising, in a sequence corresponding to amino acid positions 1 to 3093 of the amino acid sequence set forth in SEQ ID NO: 1 or a variant sequence thereof or a fragment thereof,
      at least one or more amino acid substitutions, additions, deletions, or combinations thereof in a sequence corresponding to amino acid positions 2855 to 3093, and
      a sequence having a sequence corresponding to amino acid positions 22 to 50 and 161 to 175 or a variant sequence thereof,
   (C) a polypeptide, which is a sequence corresponding to amino acid positions 1 to 2846 of the amino acid sequence set forth in SEQ ID NO: 1 or a variant sequence thereof or a fragment thereof, comprising
      a sequence corresponding to amino acid positions 22 to 50 and 161 to 175 or a variant sequence thereof,
   (D) a polypeptide comprising
      a part of amino acid positions 1 to 3518 of the amino acid sequence set forth in SEQ ID NO: 1,
      at least one or more amino acid substitutions, additions, deletions, or combinations thereof in a sequence corresponding to amino acid positions 2855 to 3093 of SEQ ID NO: 1, and
      a sequence corresponding to amino acid positions 22 to 50 and 161 to 175 of SEQ ID NO: 1 or a variant sequence thereof, (E) a polypeptide, which is a sequence corresponding to amino acid positions 1 to 2854 of the amino acid sequence set forth in SEQ ID NO: 1 or a variant sequence thereof or a fragment thereof, having
      a sequence corresponding to amino acid positions 22 to 50 and 161 to 175 or a variant sequence thereof, or (F) a polypeptide, which is a sequence corresponding to amino acid positions 1 to 268 of the amino acid sequence set forth in SEQ ID NO: 1 or a variant sequence thereof or a fragment thereof, having
      a sequence corresponding to amino acid positions 22 to 50 and 161 to 175 or a variant sequence thereof.
(5A) The polypeptide according to any of the preceding items, which is
   (a) a polypeptide, which is an amino acid sequence corresponding to amino acid positions 1 to 2854 of the amino acid sequence set forth in SEQ ID NO: 1 or a variant sequence thereof or a fragment thereof, having a sequence corresponding to amino acid positions 22 to 50 and 161 to 175 or a variant sequence thereof,
   (b) a polypeptide, which is an amino acid sequence corresponding to amino acid positions 1 to 2846 of the amino acid sequence set forth in SEQ ID NO: 1 or a variant sequence thereof or a fragment thereof, having a sequence corresponding to amino acid positions 22 to 50 and 161 to 175 or a variant sequence thereof,
   (c) a polypeptide, which is a sequence corresponding to amino acid positions 1 to 268 of the amino acid sequence set forth in SEQ ID NO: 1 or a variant sequence thereof or a fragment thereof, having a sequence corresponding to amino acid positions 22 to 50 and 161 to 175 or a variant sequence thereof, or
   (d) a polypeptide, which is a sequence corresponding to amino acid positions 1 to 257 of the amino acid sequence set forth in SEQ ID NO: 1 or a variant sequence thereof or a fragment thereof, having a sequence corresponding to amino acid positions 22 to 50 and 161 to 175 or a variant sequence thereof.
(5B) The polypeptide according to any of the preceding items, which is
   (i) a polypeptide comprising an amino acid sequence corresponding to amino acid positions 1 to 2854 of the amino acid sequence set forth in SEQ ID NO: 1 or a variant sequence thereof or a fragment thereof,
   (ii) a polypeptide comprising an amino acid sequence corresponding to amino acid positions 1 to 2846 of the amino acid sequence set forth in SEQ ID NO: 1 or a variant sequence thereof or a fragment thereof,
   (iii) a polypeptide comprising an amino acid sequence corresponding to amino acid positions 1 to 268 of the amino acid sequence set forth in SEQ ID NO: 1 or a variant sequence thereof or a fragment thereof, or
   (iv) a polypeptide comprising an amino acid sequence corresponding to amino acid positions 1 to 257 of the amino acid sequence set forth in SEQ ID NO: 1 or a variant sequence thereof or a fragment thereof.
(5C) The polypeptide according to any of the preceding items, which is an amino acid sequence corresponding to amino acid positions 1 to 2854 of the amino acid sequence set forth in SEQ ID NO: 1 or a variant sequence thereof or a fragment thereof.
(5D) The polypeptide according to any of the preceding items, which is an amino acid sequence corresponding to amino acid positions 1 to 2846 of the amino acid sequence set forth in SEQ ID NO: 1 or a variant sequence thereof or a fragment thereof.
(5D) The polypeptide according to any of the preceding items, which is an amino acid sequence corresponding to amino acid positions 1 to 268 of the amino acid sequence set forth in SEQ ID NO: 1 or a variant sequence thereof or a fragment thereof.
(5E) The polypeptide according to any of the preceding items, which is an amino acid sequence corresponding to amino acid positions 1 to 257 of the amino acid sequence set forth in SEQ ID NO: 1 or a variant sequence thereof or a fragment thereof.
(6)The polypeptide according to any of the preceding items, consisting of an amino acid sequence corresponding to amino acid positions 1 to 2854 of the amino acid sequence set forth in SEQ ID NO: 1.
(7)The polypeptide according to any of the preceding items, consisting of an amino acid sequence corresponding to amino acid positions 1 to 2846 of the amino acid sequence set forth in SEQ ID NO: 1.
(8)The polypeptide according to any of the preceding items, consisting of an amino acid sequence corresponding to amino acid positions 1 to 268 of the amino acid sequence set forth in SEQ ID NO: 1.
(9)The polypeptide according to any of the preceding items, consisting of an amino acid sequence corresponding to amino acid positions 1 to 257 of the amino acid sequence set forth in SEQ ID NO: 1.
(10) The polypeptide according to any of the preceding items, wherein the variant sequence comprises an alteration by a conservative substitution.
(11) The polypeptide according to any of the preceding items, which is derived from an *Acinetobacter* bacterium.
(12) The polypeptide according to any of the preceding items, wherein the *Acinetobacter* bacterium is of an *Acinetobacter* sp. Tol5 strain.
(13) The polypeptide according to any of the preceding items, further comprising an amino acid sequence that is different from SEQ ID NO: 1.
(14) The polypeptide according to any of the preceding items, wherein the amino acid sequence that is different is fused with the fragment.
(15) A complex of the polypeptide according to any of the preceding items and a functionality-imparting entity.
(16) A composition for use in causing adhesion and/or immobilization of a first subject to a second subject without self-aggregation, comprising the polypeptide according to any of the preceding items or the complex according to any of the preceding items.
(17) The composition according to any of the preceding items for use in causing monolayer adhesion of the first subject to the second subject.
(18) The composition according to any of the preceding items, wherein the first subject and the second subject are the same.
(19) The composition according to any of the preceding items, wherein the first subject and the second subject are different.
(20) The composition according to any of the preceding items, wherein at least one of the first subject or the second subject is a functionality-imparting entity.
(21) The composition according to any of the preceding items, wherein the functionality-imparting entity is a peptide and/or a protein.
(22) The composition according to any of the preceding items, wherein the functionality-imparting entity is streptavidin, neutral avidin, or a variant thereof.
(23) The composition according to any of the preceding items, wherein the functionality-imparting entity is a cell.
(24) The composition according to any of the preceding items, wherein the second subject is a support.
(25) The composition according to any of the preceding items, wherein the support is a liposome.
(26) A composition for use in causing adhesion and/or immobilization of a first subject to a second subject without self-aggregation, comprising the polypeptide according to any of the preceding items or the complex according to any of the preceding items, wherein the first subject is the peptide comprising the different amino acid sequence or the functionality-imparting entity.
(27) A method for use in causing adhesion and/or immobilization of a first subject to a second subject, comprising causing the polypeptide according to any of the preceding items or the complex according to any of the preceding items to act on the first and/or second subject.
(28) The method according to any of the preceding items for use in causing monolayer adhesion of the first subject to the second subject.
(29) The method according to any of the preceding items, wherein the first subject and the second subject are the same.
(30) The method according to any of the preceding items, wherein the first subject and the second subject are different.
(31) The method according to any of the preceding items, wherein at least one of the first subject or the second subject is a functionality-imparting entity.
(32) The method according to any of the preceding items, wherein the functionality-imparting entity is a peptide and/or a protein.
(33) The method according to any of the preceding items, wherein the functionality-imparting entity is streptavidin, neutral avidin, or a variant thereof.
(34) The method according to any of the preceding items, wherein the functionality-imparting entity is a cell.
(35) The method according to any of the preceding items, wherein the second subject is a support.
(36) The method according to any of the preceding items, wherein the support is a liposome.
(37) A method for use in causing adhesion and/or immobilization of the peptide comprising the different amino acid sequence or the functionality-imparting entity to a second subject without self-aggregation, comprising causing the polypeptide according to any of the preceding items or the complex according to any of the preceding items to act on the second subject.
(38) A support on which the polypeptide according to any of the preceding items or the complex according to any of the preceding items is immobilized.
(39) The support according to any of the preceding items, wherein the support is selected from the group consisting of a lipid bilayer structure, glass, silica, silicon, ceramic, silicon dioxide, plastic, metal, titanium, acrylic material, and naturally-occurring and synthetic polymers.
(40) Use of the polypeptide according to any of the preceding items or the complex according to any of the preceding items for causing adhesion and/or immobilization of a first subject to a second subject without self-aggregation.
(41) The use according to any of the preceding items, wherein the use is use for causing monolayer adhesion of the first subject to the second subject.
(42) The use according to any of the preceding items, wherein the first subject and the second subject are the same.
(43) The use according to any of the preceding items, wherein the first subject and the second subject are different.
(44) The use according to any of the preceding items, wherein at least one of the first subject or the second subject is a functionality-imparting entity.
(45) The use according to any of the preceding items, wherein the functionality-imparting entity is a peptide and/or a protein.
(46) The use according to any of the preceding items, wherein the functionality-imparting entity is streptavidin, neutral avidin, or a variant thereof.
(47) The use according to any of the preceding items, wherein the functionality-imparting entity is a cell.
(48) The use according to any of the preceding items, wherein the second subject is a support.
(49) The use according to any of the preceding items, wherein the support is a liposome.
(50) Use of the polypeptide according to any of the preceding items or the complex according to any of the preceding items for causing adhesion and/or immobilization of a first subject to a second subject without self-aggregation, wherein the first subject is the peptide comprising the different amino acid or the functionality-imparting entity.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Use of the present disclosure has enabled immobilization and/or adhesion of an adhesive polypeptide (including fusion polypeptide) and/or a complex of an adhesive polypeptide and a functionality-imparting entity of the present disclosure without self-aggregation. In one aspect, the present disclosure has enabled immobilization and/or adhesion of an adhesive polypeptide (including fusion polypeptide) and a functionality-imparting entity on a membrane of a liposome. Thus, the adhesive polypeptide of the present disclosure is useful for a separation process in pharmaceutical/chemical product industries, regenerative medicine, cellular engineering, medical engineering, and biosensors, as well as material engineering such as surface modification and designing of functional materials.

### [Brief Description of Drawings]

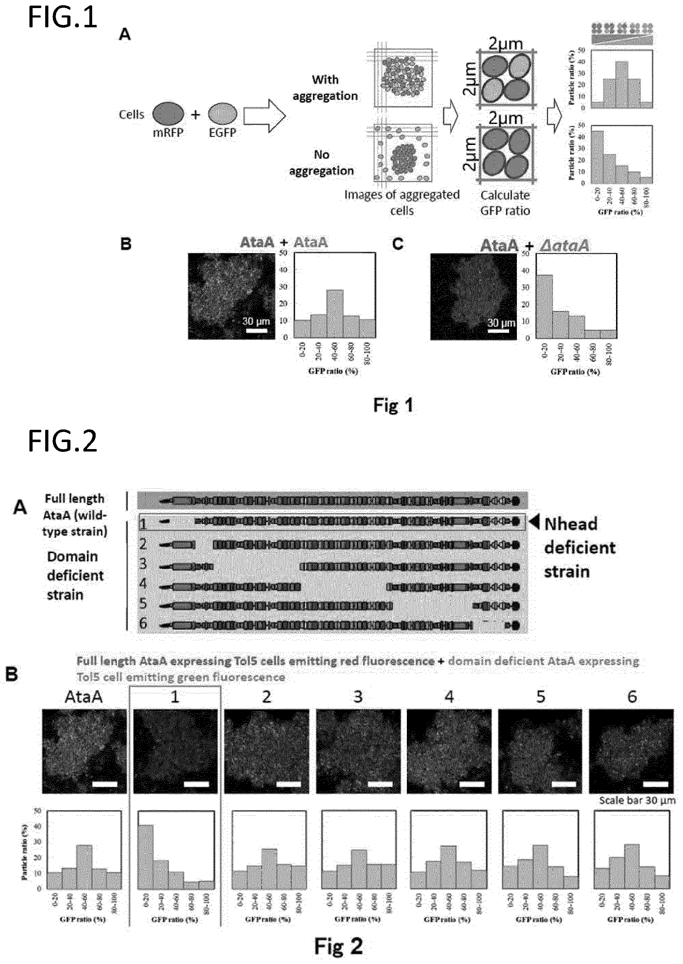

[Figure 1] Figure **1** shows analysis of aggregation of cells through observation under a microscope. Figure **1A** shows a model system for aggregation analysis. In Figure **1A****,** an ellipse indicates a cell. A dark ellipse indicates a cell expressing mRFP (red fluorescent protein). A light ellipse indicates a cell expressing EGFP (green fluorescent protein). The top side of the center of Figure **1A** shows an aggregation pattern of cells when both cells have an aggregation action. The bottom row of the center shows an aggregation pattern of cells when only mRFP expressing cells have an aggregation action while EGFP expressing cells do not have an aggregation action. The third part from the left in Figure **1A** shows a pattern of abundance of cells when each aggregate of cells is divided into a 2 µm section. The right most side of Figure **1A** shows graphs counting each pattern of abundance of cells based on the ratio of cells emitting fluorescence of GFP. In the graphs, the horizontal axis represents the ratio of cells emitting fluorescence of GFP, showing that fluorescence of GFP was observed in, in order from the left, 0 to 20%, 20 to 40%, 40 to 60%, 60 to 80%, or 80 to 100% of cells. The vertical axis indicates the ratio of the number of sections found to have each ratio of cells emitting fluorescence of GFP.
Figure **1B** shows an image of a cell aggregate formed by mixing an *Acinetobacter* sp. Tol5 strain expressing mRFP and a normal AtaA protein and an *Acinetobacter* sp. Tol5 strain expressing EGFP and a normal AtaA protein, and a histogram of the ratio of cells expressing EGFP in a small section.
Figure **1C** shows an image of a cell aggregate formed by mixing an *Acinetobacter* sp. Tol5 strain expressing mRFP and a normal AtaA protein and a ΔAtaA *Acinetobacter* sp. Tol5 strain expressing EGFP, and a histogram of the ratio of cells expressing EGFP in a small section.
[Figure 2] Figure **2** shows an aggregation experiment using a full length AtaA protein or a partially deficient strain. Figure **2A** shows the domain structure of, in order from the top, a full length AtaA protein, (1) AtaA with a structure that is deficient of Nhead, (2) AtaA with a structure that is deficient of a part of Nstalk, (3) AtaA with a structure that is deficient of a part of Nstalk, (4) AtaA with a structure that is deficient of a part of Nstalk, (5) AtaA with a structure that is deficient of Chead in addition to a part of Nstalk, and (6) AtaA with a structure that is deficient of the majority of Cstalk (while the FGG domain is preserved) . Figure **2B** shows images of a cell aggregate formed by mixing an *Acinetobacter* sp. Tol5 strain expressing mRFP and a normal AtaA protein and an *Acinetobacter* sp. Tol5 strain expressing EGFP and a normal AtaA protein or the 6 types of partially deficient forms in Figure **2A****,** and histograms of the ratio of cells expressing EGFP in a small section.

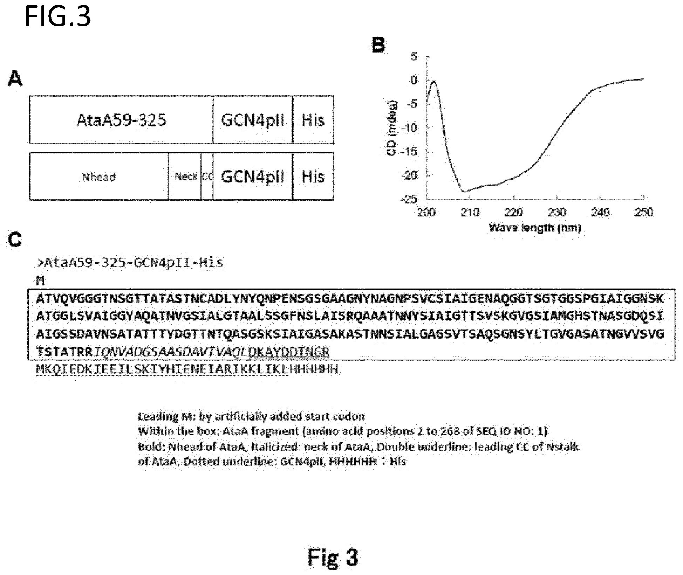

[Figure 3] Figure **3** shows that an Nhead recombinant protein has a secondary structure. Figure **3A** is a diagram that clearly shows, in order from the top, the construct design of an Nhead recombinant protein and the domain structure of an AtaA fragment portion of an Nhead recombinant protein. Figure **3B** shows results of measurement of CD spectra of an Nhead recombinant protein. In Figure **3B****,** the horizontal axis of the graph indicates wavelength (nm), and the vertical axis indicates CD (mdeg). Figure **3C** shows the amino acid sequence of an Nhead recombinant protein (AtaA₅₉₋₃₂₅-GCN4pII-His) and detailed explanation thereof.

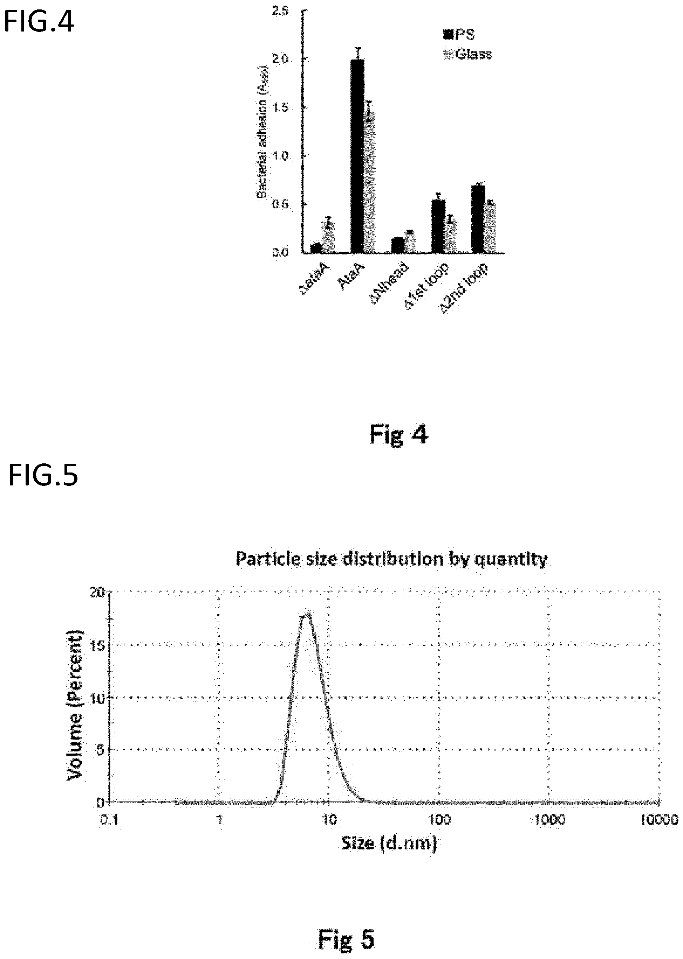

[Figure 4] Figure **4** shows results of measuring an adhesive property of microorganisms by using a mutant deficient of a loop structure within the Nhead domain. The axis in the graph indicates results from using, in order from the left, AtaA deficient strain (ΔataA), wild-type (AtaA), ΔNhead deficient strain, Δ1st loop deficient strain, and Δ2nd loop deficient strain. Black bar graphs correspond to adhesion to a polystyrene plate, and gray bar graphs correspond to adhesion to a glass plate. The vertical axis of the graph indicates an adhesive property of microorganisms measured by A₅₉₀.
[Figure 5] Figure **5** shows the dispersion and self-aggregation property of *E. coli* recombinant Nhead. The horizontal axis of the graph indicates the size of purified Nhead (d. nm), and the vertical axis indicates the volume of purified Nhead (percent).

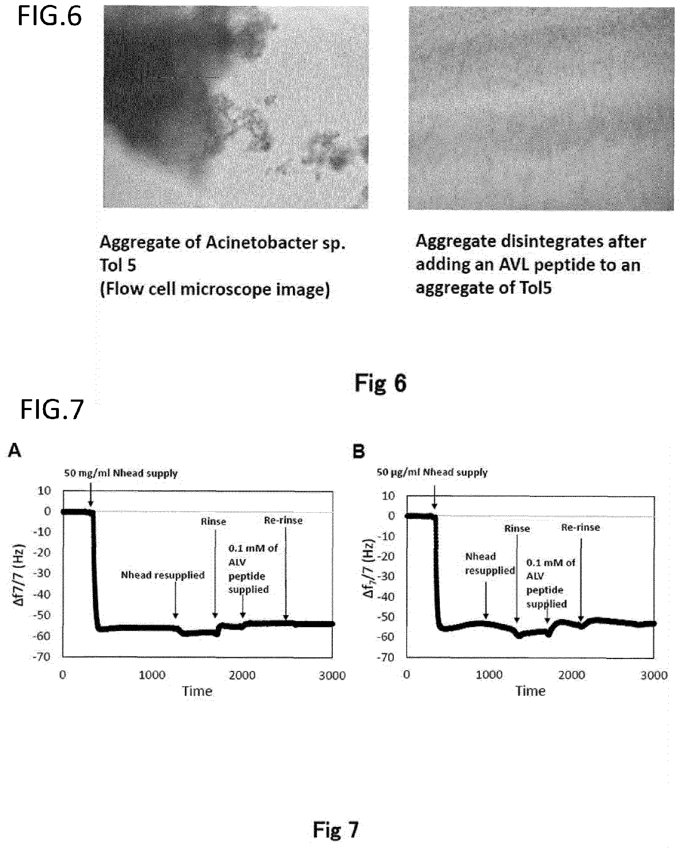

[Figure 6] Figure **6** shows microscope pictures of an aggregate of an *Acinetobacter* sp. Tol5 strain. The left side shows a microscope picture of an aggregate of an *Acinetobacter* sp. Tol5 strain, and the right side shows a microscope picture from when an AVL peptide was added to an *Acinetobacter* sp. Tol5 stain.
[Figure 7] Figure **7** shows an adhesive property of *E. coli* recombinant Nhead measured by QCM-D. Figure **7A** shows adhesion to a polystyrene sensor. Figure **7B** shows adhesion to a silica sensor. In each graph, the vertical axis indicates the amount of adhesion by a Δf7/7 (Hz) value, and the horizontal axis indicates time. Each arrow in the graphs indicates, in order from the left, time at which 50 µg/ml of Nhead was added, time at which Nhead was added again, time at which the sample was rinsed, time at which 0.1 mM of AVL peptide was added, and time at which the sample was rinsed again.

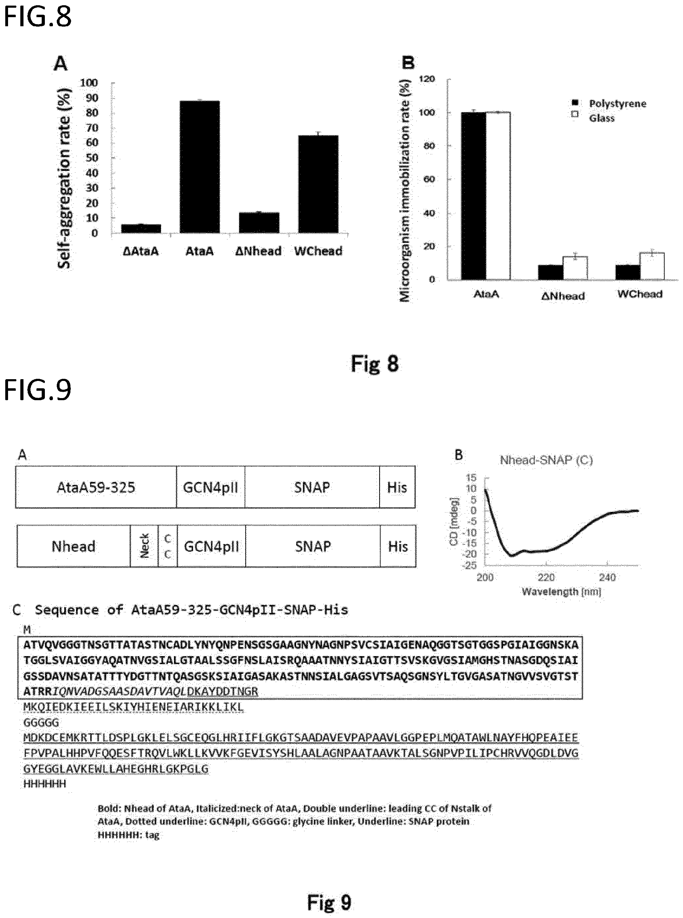

[Figure 8] Figure **8** shows the self-aggregation property and microorganism immobilization rates of a bacterial strain expressing an AtaA variant of WChead prepared from substituting Nhead of full length AtaA with Chead. Figure **8A** shows a self-aggregation property of a strain that does not express AtaA (ΔAtaA), wild-type strain (AtaA), strain that expresses AtaA deficient of Nhead (ΔNhead), and strain that expresses an AtaA variant of WChead (WChead). Figure **8B** shows the immobilization rates of the wild-type strain (AtaA), strain that expresses AtaA deficient of Nhead (ΔNhead), and strain that expresses an AtaA variant of WChead (WChead) to a polystyrene surface or glass surface.
[Figure 9] Figure **9** shows that an Nhead-SNAP fusion peptide has a secondary structure. Figure **9A** is a diagram that clearly shows, in order from the top, the construct design of an Nhead-SNAP fusion peptide and domain structure of the AtaA fragment portion of an Nhead-SNAP fusion peptide. Figure **9B** shows results of measuring CD spectra of an Nhead-SNAP fusion peptide. In Figure **9B****,** the horizontal axis of the graph indicates wavelength (nm), and the vertical axis indicates CD (mdeg). Figure **9C** shows the amino acid sequence of an Nhead-SNAP fusion peptide (AtaA₅₉₋₃₂₅-GCN4pII-SNAP-His) and detailed explanation thereof.

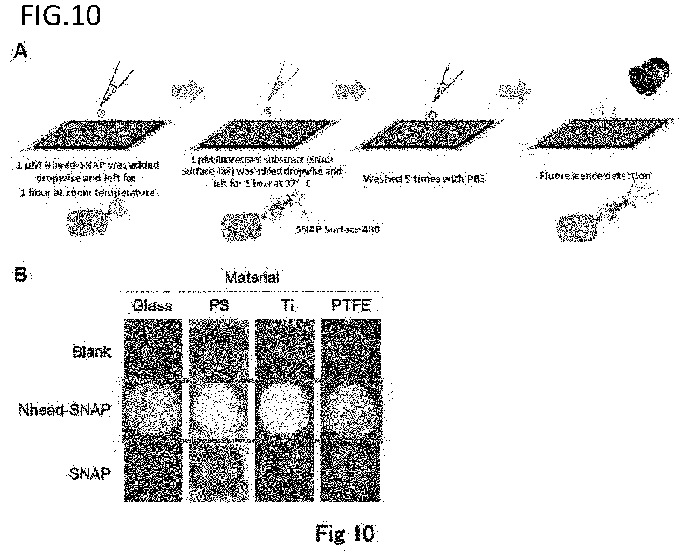

[Figure 10] Figure **10** shows immobilization of an Nhead-SNAP fusion peptide onto various material surfaces. Figure **10A** shows a model for testing immobilization of an Nhead-SNAP fusion peptide onto various material surfaces with a fluorescent molecule substrate of a SNAP protein fused to Nhead, corresponding to the steps of, in order from the left, adding an Nhead-SNAP fusion peptide dropwise onto the material surface, adding a fluorescent molecule substrate dropwise, washing, and measuring fluorescence. Figure **10B** shows fluorescence when an Nhead-SNAP fusion peptide or SNAP protein alone adhered to various materials. The columns in the picture show, in order from the left, glass, polystyrene, titanium, and polytetrafluoroethylene resin material. The rows in the picture show fluorescence when, in order from the top, no protein is adhering thereto, an Nhead-SNAP fusion peptide is adhering thereto, and a SNAP protein is adhering thereto.

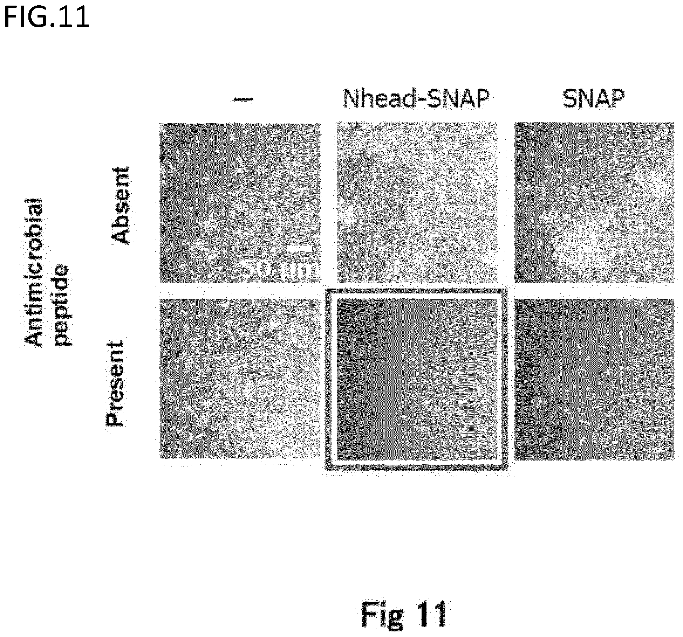

[Figure 11] Figure **11** shows the antimicrobial property of the surface when an antimicrobial peptide CAP18 with an addition of a benzylguanyl (BG) group was immobilized on a polystyrene surface via an Nhead-SNAP fusion peptide. The columns of the picture indicate, in order from the left, a surface with nothing adhering thereto, a surface with an Nhead-SNAP fusion peptide adhering thereto, and surface with a SNAP protein adhering thereto. The rows of the picture indicate, in order from the top, an antimicrobial peptide was not allowed to act on the surface (not coated with an antimicrobial peptide) and an antimicrobial peptide was allowed to act on the surface (coated with an antimicrobial peptide). The portions that appear white in each picture indicate fluorescence from Pseudomonas aeruginosa (fluorescently stained) adhering to the material surface. Less white portions indicates that microorganisms are not adhering from sterilization, and thus a higher antimicrobial property. The picture in the middle of the bottom row hardly has any white portion compared to other pictures and is found to have a high antimicrobial effect on Pseudomonas aeruginosa.

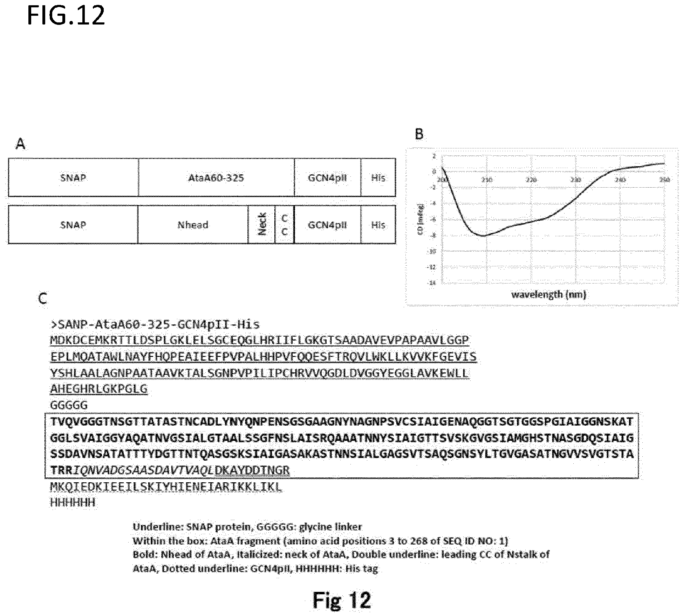

[Figure 12] Figure **12** shows that a SNAP-Nhead fusion peptide with a SNAP protein at the N-terminus has a secondary structure. Figure **12A** is a diagram that clearly shows, in order from the top, the construct design of a SNAP-Nhead fusion peptide and domain structure of the AtaA fragment portion of a SNAP-Nhead fusion peptide. Figure **12B** shows results of measuring CD spectra of a SNAP-Nhead fusion peptide. In Figure **12B****,** the horizontal axis of the graph indicates wavelength (nm), and the vertical axis indicates CD (mdeg). Figure **12C** shows the amino acid sequence of a SNAP-Nhead fusion peptide (SNAP-AtaA₆₀₋₃₂₅-GCN4pII-His) and detailed explanation thereof.

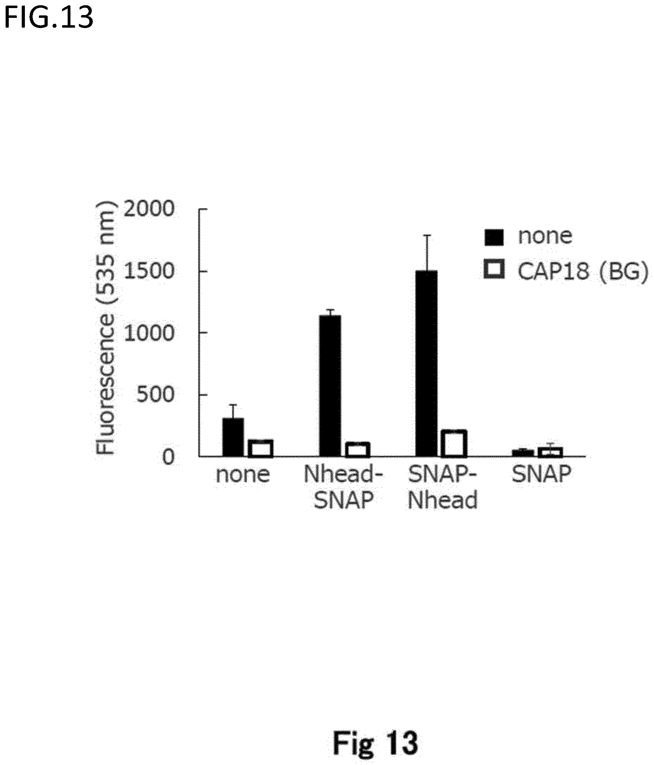

[Figure 13] Figure **13** shows a comparison of a SNAP protein fused to the N-terminus side of Nhead (SNAP-Nhead) and that fused to the C-terminus side (Nhead-SNAP). The graph shows a comparison of adhesive properties between an Nhead-SNAP fusion peptide and a SNAP-Nhead fusion peptide. The vertical axis of the graph indicates the fluorescence intensity at 535 nm, and the horizontal axis indicates, in order from the left, no adhesion, adhesion of an Nhead-SNAP fusion peptide, adhesion of a SNAP-Nhead fusion peptide, and adhesion of a SNAP protein. The black bars in the graph indicate fluorescence intensity from no addition of BG-lated CAP18 (BG), and white bars indicate fluorescence intensity from addition of BG-lated CAP18 (BG). The difference between black and white bars indicates an amount of fluorescent substrate that can no longer adhere due to adhesion of BG-lated CAP18, i.e., an amount corresponding to the amount of adhesion of BG-lated CAP18.

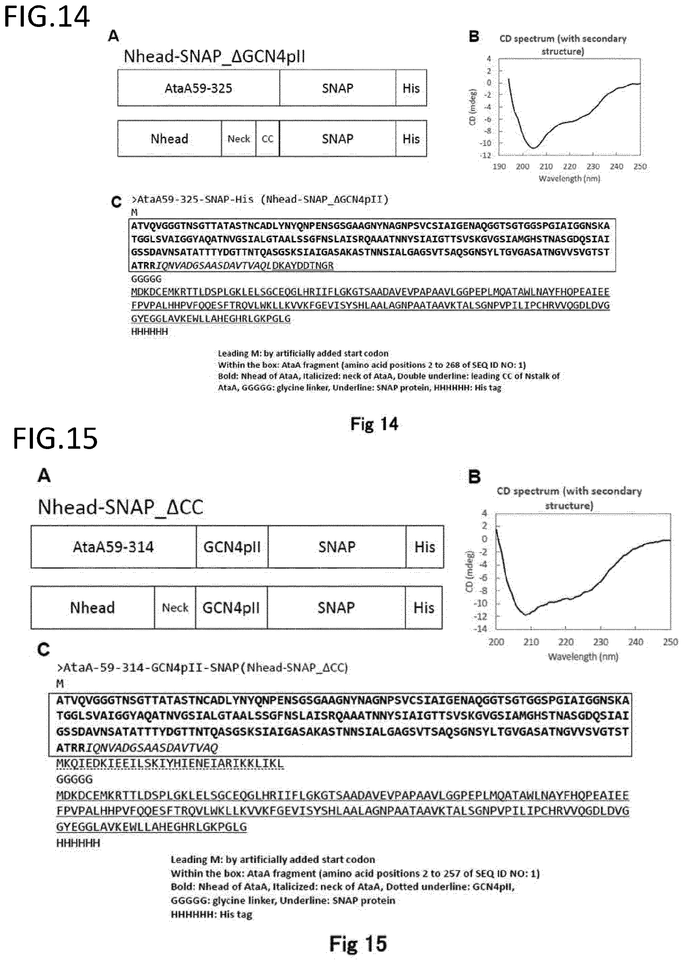

[Figure 14] Figure **14** shows that an Nhead-SNAP fusion peptide which does not use a GCN4pII tag has a secondary structure. Figure **14A** is a diagram that clearly shows, in order from the top, the construct design of an Nhead-SNAP_ΔGCN4pII fusion peptide and domain structure of the AtaA fragment portion of an Nhead-SNAP_ΔGCN4pII fusion peptide. Figure **14B** shows results of measuring CD spectra of an Nhead-SNAP_ΔGCN4pII fusion peptide. In Figure **14B****,** the horizontal axis of the graph indicates wavelength (nm), and the vertical axis indicates CD (mdeg). Figure **14C** shows the amino acid sequence of an Nhead-SNAP_ΔGCN4pII fusion peptide (AtaA₅₉₋₃₂₅-SNAP-His) and detailed explanation thereof.
[Figure 15] Figure **15** shows that a (ΔCC)Nhead-SNAP fusion peptide with a tip coiled coil region at the N-terminus of Nstalk deleted has a secondary structure. Figure **15A** is a diagram that clearly shows, in order from the top, the construct design of an Nhead-SNAP_ΔCC fusion peptide with a deleted coiled coil, which is a part of an FGG domain at the tip of the N-terminus of Nstalk, and domain structure of the AtaA fragment portion of an Nhead domain-SNAP_ΔCC fusion peptide. Figure **15B** shows results of measuring CD spectra of an Nhead-SNAP_ΔCC fusion peptide. In Figure **15B****,** the horizontal axis of the graph indicates wavelength (nm), and the vertical axis indicates CD (mdeg). Figure **15C** shows the amino acid sequence of an Nhead-SNAP_ΔCC fusion peptide (AtaA₅₉₋₃₁₄-GCN4pII-SNAP-His) and detailed explanation thereof.

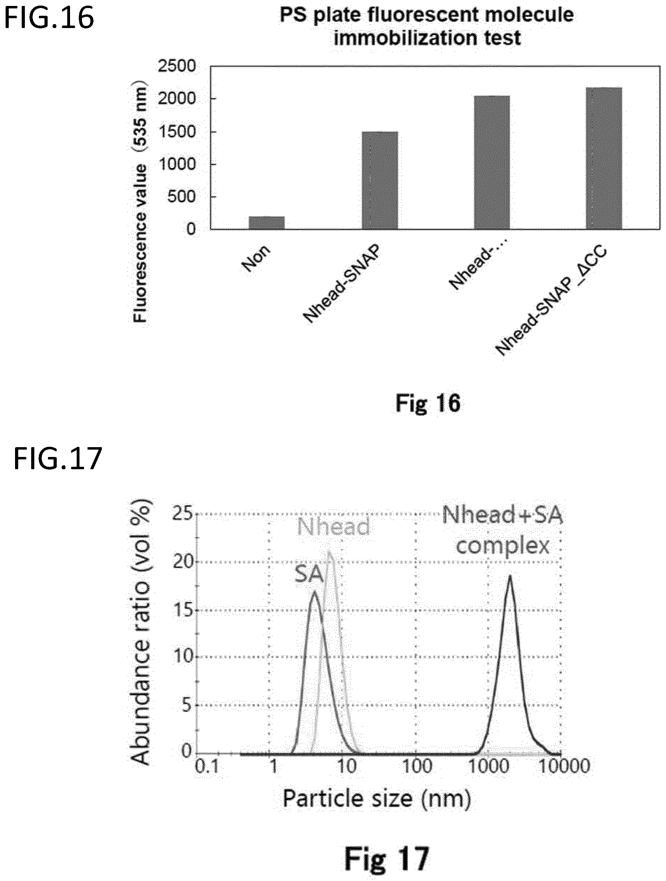

[Figure 16] Figure **16** shows results of immobilization tests for various Nhead-SNAP fusion peptides shown in Figures **9****,** **14,** and **15****.** The results are shown from measuring the amount of immobilization of fluorescent molecule substrate from the fluorescence intensity of a fluorescent molecule that forms a covalent bond with a SNAP protein, which is a substrate of the SNAP protein. The graph shows the results of fluorescent molecule immobilization tests from coating a polystyrene (PS) plate with, in order from the left, Non (only added a fluorescent molecule substrate with no coating of protein), Nhead-SNAP fusion peptide, Nhead-SNAP_ΔGCN4pII fusion peptide, and Nhead-SNAP_ΔCC fusion peptide. The vertical axis of the graph indicates the value of fluorescence (535 nm).
[Figure 17] Figure **17** shows that *E. coli* recombinant Nhead and streptavidin form a complex. The graph shows results of measuring the particle size of Nhead or streptavidin (SA) alone or a mixture thereof by dynamic light scattering (DLS). The horizontal axis of the graph indicates the particle size, and the vertical axis indicates the abundance ratio of particles by volume ratio. The lines with a peak near the particle size of 10 nm correspond to the measurement results for Nhead alone and SA alone, and the line with a peak at a particle size exceeding 1000 nm corresponds to the measurement results for the complex of Nhead and SA in the graph.

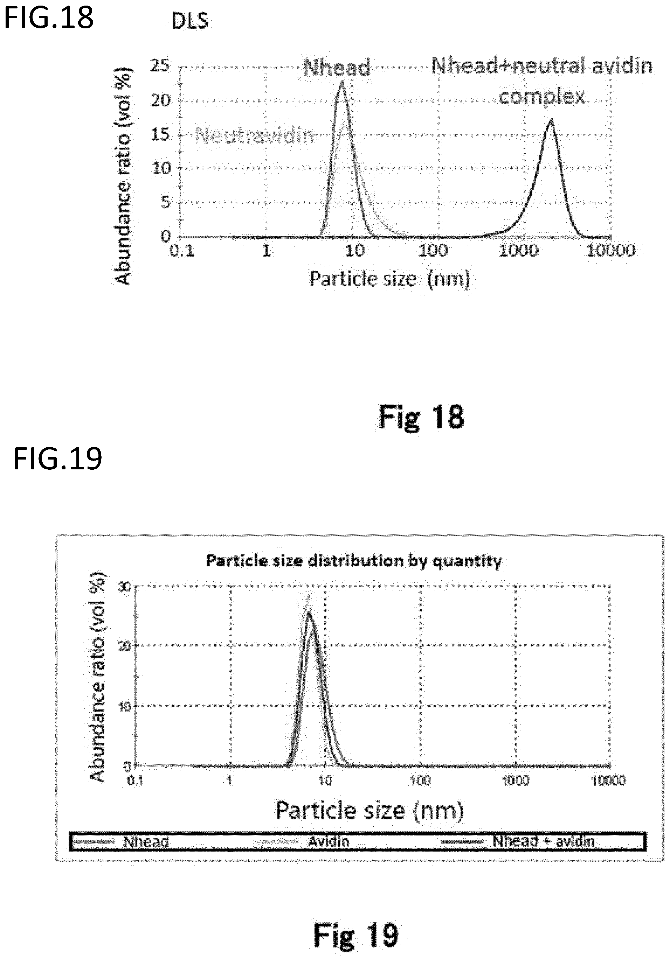

[Figure 18] Figure **18** shows that *E. coli* recombinant Nhead binds to neutral adivin. The graph shows results of measuring the particle sizes of Nhead alone, neutral avidin alone, and mixture thereof by dynamic light scattering (DLS). The horizontal axis of the graph indicates the particle size (nm), and the vertical axis indicates the abundance ratio (volume %) of each particle. The horizontal axis of the graph indicates the particle size, and the vertical axis indicates the abundance ratio of particles by volume ratio. The lines with a peak near the particle size of 10 nm correspond to the measurement results for Nhead alone and neutral avidin alone, and the line with a peak at a particle size exceeding 1000 nm corresponds to the measurement results for the complex of Nhead and neutral avidin in the graph.
[Figure 19] Figure **19** is a result of a comparative example showing that *E. coli* recombinant Nhead does not bind to or form a complex with avidin. The graph shows results of measuring the particles sizes of Nhead alone, avidin alone, and mixture thereof by dynamic light scattering (DLS). The horizontal axis of the graph indicates the particle size, and the vertical axis indicates the abundance ratio of particles by volume ratio.

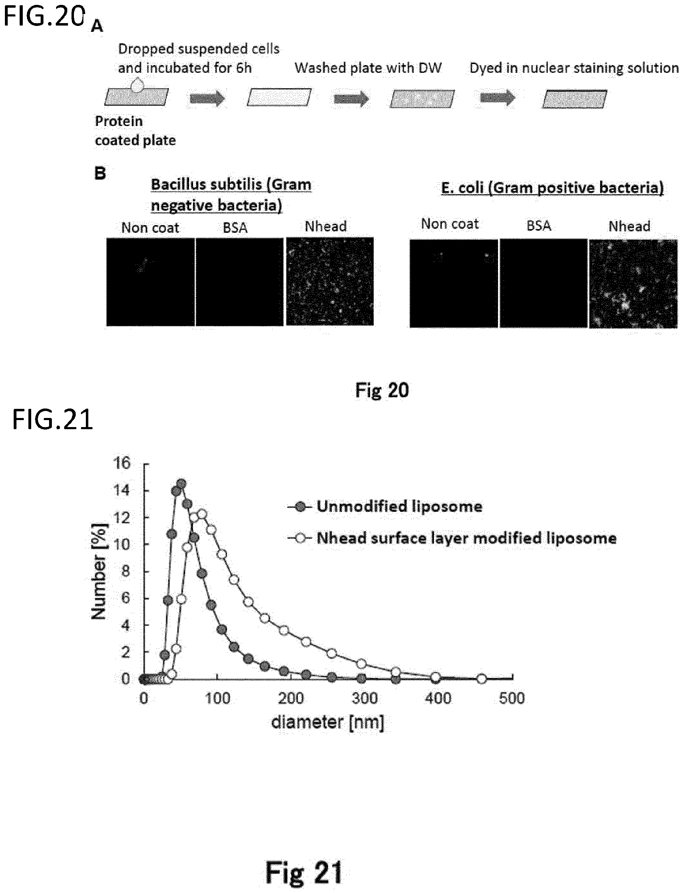

[Figure 20] Figure **20** shows immobilization of bacteria onto a plate coated with *E. coli* recombinant Nhead on the surface. Figure **20A** shows an experimental system for detecting immobilization of bacteria. Figure **20B** shows pictures of immobilized Bacillus subtilis on the left side, and pictures of immobilized *E. coli* on the right side. For each bacteria, the pictures show pictures from instances where, in order from left, no coating was applied, BSA coating was applied, and Nhead coating was applied. In each picture, fluorescently stained bacterial cells appear white.
[Figure 21] Figure **21** shows analysis of particles of liposomes prepared by immobilizing an Nhead-SNAP fusion peptide onto the surface layer (Nhead surface layer modified liposomes). In Figure **21****,** the vertical axis of the graph indicates the number based ratio (%) for each diameter of liposome, and the horizontal axis indicates the diameter of liposome.

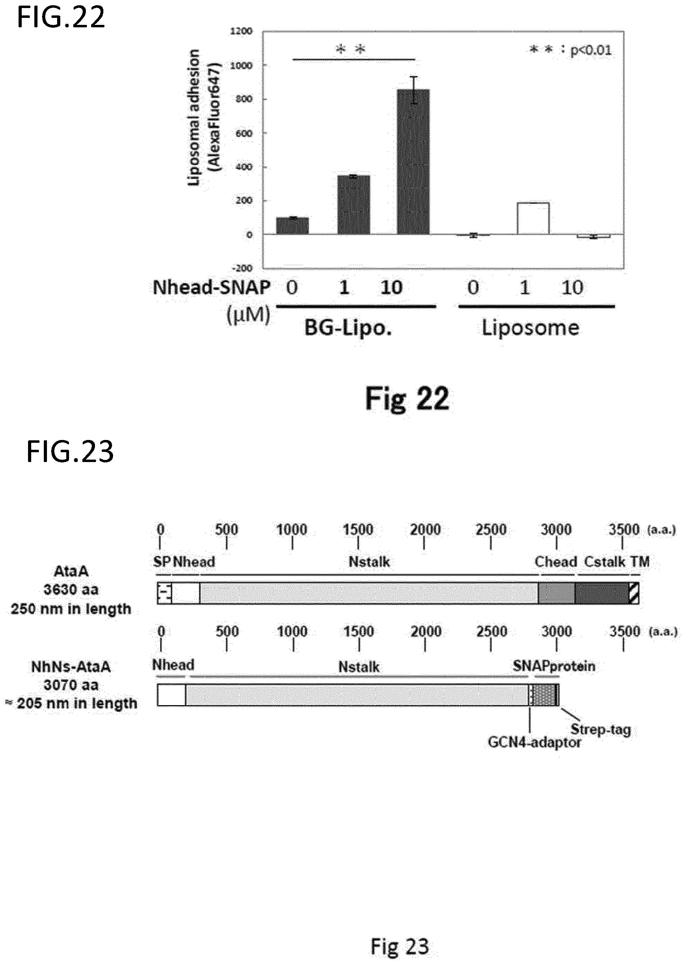

[Figure 22] Figure **22** shows immobilization of a liposome prepared by immobilizing an Nhead-SNAP fusion peptide onto the surface layer (Nhead surface layer modified liposome). In Figure **22****,** the vertical axis of the graph indicates the fluorescence intensity of Alexa Fluor 647, and the horizontal axis shows results for Nhead surface layer modified liposomes mediated by a benzylguanyl (BG) group when 0 µM, 1 µM, or 10 µM of Nhead-SNAP fusion peptide was added, respectively, and a normal liposome without modification.
[Figure 23] Figure **23** shows the construct design of an *E*. *coli* recombinant Nhead Nstalk (NhNs)-SNAP fusion peptide. The top row shows the full length of an ApaA protein, and the bottom row shows an NhNs-SNAP fusion peptide. The full length sequence on the top row shows the domain group structure of an AtaA protein, corresponding to, in order from the left, SP (signal peptide), Nhead, Nstalk, Chead, Cstalk, and TM (membrane anchor) domain. The bottom row shows the configuration of a fusion peptide, corresponding to, in order from the left, Nhead, Nstalk, GCN4 adaptor, SNAP protein, and Strep tag.

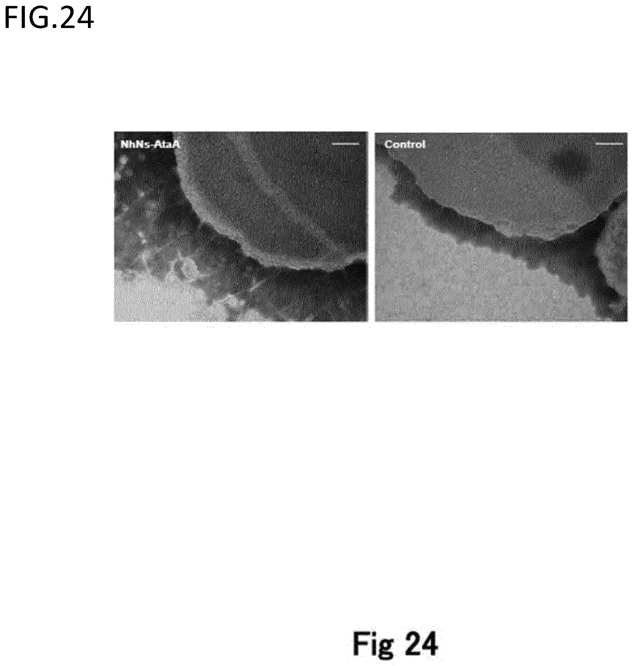

[Figure 24] Figure **24** shows microscope pictures of an NhNs fiber surface layer modified liposome and a normal liposome as a control. The left side is a picture of an NhNs fiber surface layer modified liposome, and the right side is a picture of an unmodified normal liposome (control).

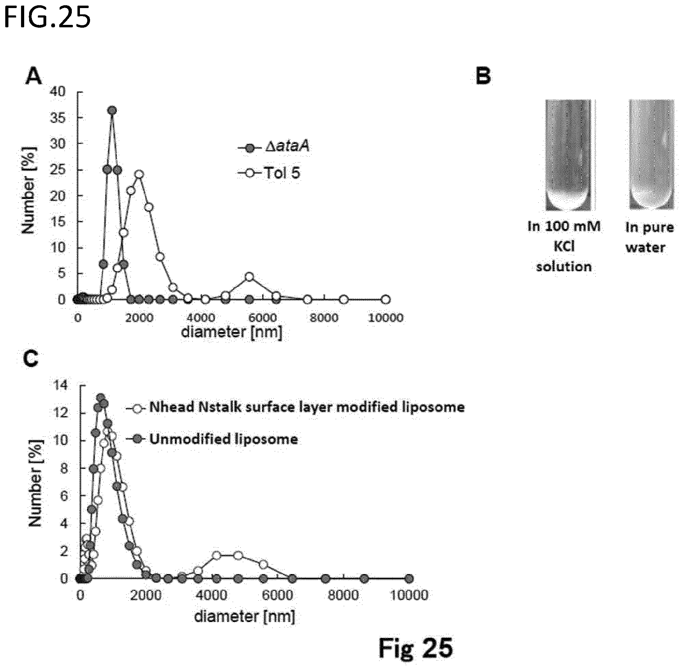

[Figure 25] Figure **25** shows results of analyzing the particle shape of an NhNs fiber surface layer modified liposome by DLS. Figure **25A** shows results of analyzing an *Acinetobacter* sp. Tol5 strain and ΔataA mutant strain suspended in pure water by DLS for comparison. White dots indicate results for the *Acinetobacter* sp. Tol5 strain, and the gray dots indicate results for the ΔataA mutant strain. Figure **25B** is a diagram showing that Tol5 strain cells do not self-aggregate in pure water. Figure **25C** shows results of analyzing an NhNs fiber surface layer modified liposome and unmodified liposome by DLS. White dots indicate results for the NhNs fiber surface layer modified liposome, and the gray dots indicate results for the unmodified liposome.

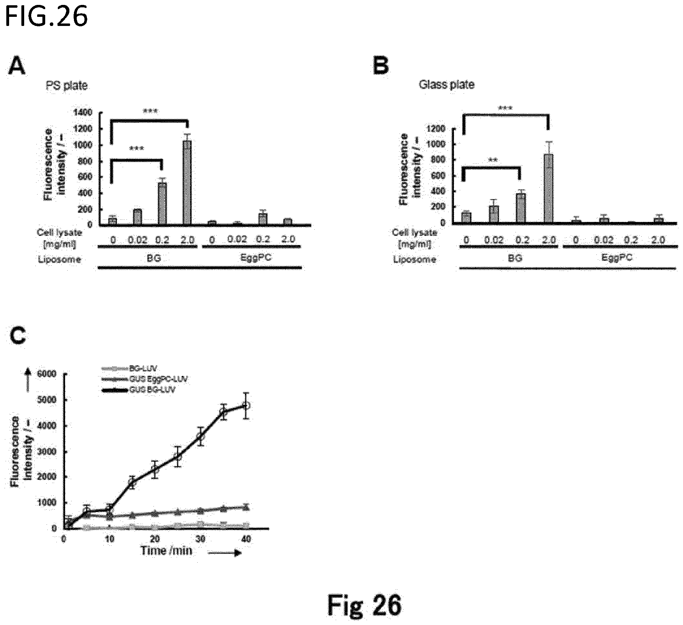

[Figure 26] Figure **26** shows an adhesion assay using an NhNs fiber surface layer modified liposome. Figure **26A** shows results of an experiment for adhesion of a liposome to a polystyrene plate. The left side shows results of adhesion of an NhNs fiber modified liposome by using a benzylguanyl group (BG) liposome, and the right side shows results of adhesion of a control unmodified liposome by using an egg phosphatidylcholine (PC) liposome. Figure **26B** shows results of an experiment for adhesion of a liposome to a glass plate. The left side shows results of adhesion of an NhNs fiber surface layer modified liposome, and the right side shows results of adhesion of a control unmodified liposome.
Figure **26C** shows results of an enzymatic reaction test using an NhNs fiber modified liposome, which encapsulates β glucuronidase (GUS) and is immobilized onto a polystyrene plate. In the graph, the line indicating data with circles corresponds to data for an NhNs fiber surface layer modified liposome encapsulating GUS, and the line indicating data with triangles corresponds to data for an unmodified liposome encapsulating GUS, and the line indicating data with squares corresponds to data for an NhNs fiber surface layer modified liposome free of GUS.

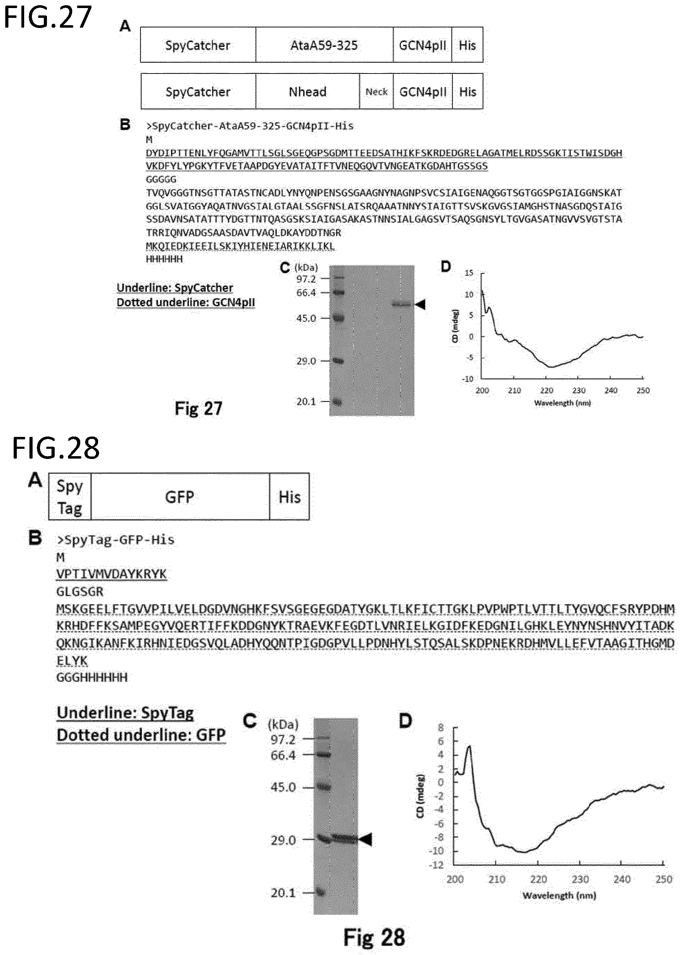

[Figure 27] Figure **27** shows that a SpyCatcher-Nhead fusion peptide fused to SpyCatcher has a secondary structure. Figure **27A** is a diagram that clearly shows, in order from the top, the construct design of a SpyCatcher-Nhead fusion peptide and domain structure of the AtaA fragment portion of a SpyCatcher-Nhead fusion peptide. Figure **27B** shows the amino acid sequence of a SpyCatcher-Nhead fusion peptide (SpyCatcher-AtaA₅₉₋₃₂₅-GCN4pII-His) and detailed explanation thereof. Figure **27C** shows results of CBB staining of a SpyCatcher-Nhead fusion peptide. In Figure **27C****,** the left side shows the molecular weight, and the arrow on the right side indicates the molecular weight of a SpyCatcher-Nhead fusion peptide. Figure **27D** shows results of measuring the CD spectra of a SpyCatcher-Nhead fusion peptide. In Figure **27D****,** the horizontal axis of the graph indicates wavelength (nm), and the vertical axis indicates CD (mdeg).
[Figure 28] Figure **28** shows that a SpyTag-GFP fusion peptide fused to SpyTag has a secondary structure. Figure **28A** shows the construct design of SpyTag-GFP. Figure **28B** shows the amino acid sequence of a SpyTag-GFP fusion peptide (SpyTag-GFP-His) and detailed explanation thereof. Figure **28C** shows results of CBB staining of a SpyTag-GFP fusion peptide. In Figure **28C****,** the left side shows the molecular weight, and the arrow on the right side indicates the molecular weight of a SpyTag-GFP fusion peptide. Figure **28D** shows results of measuring the CD spectra of a SpyTag-GFP fusion peptide. In Figure **28D****,** the horizontal axis of the graph indicates wavelength (nm), and the vertical axis indicates CD (mdeg).

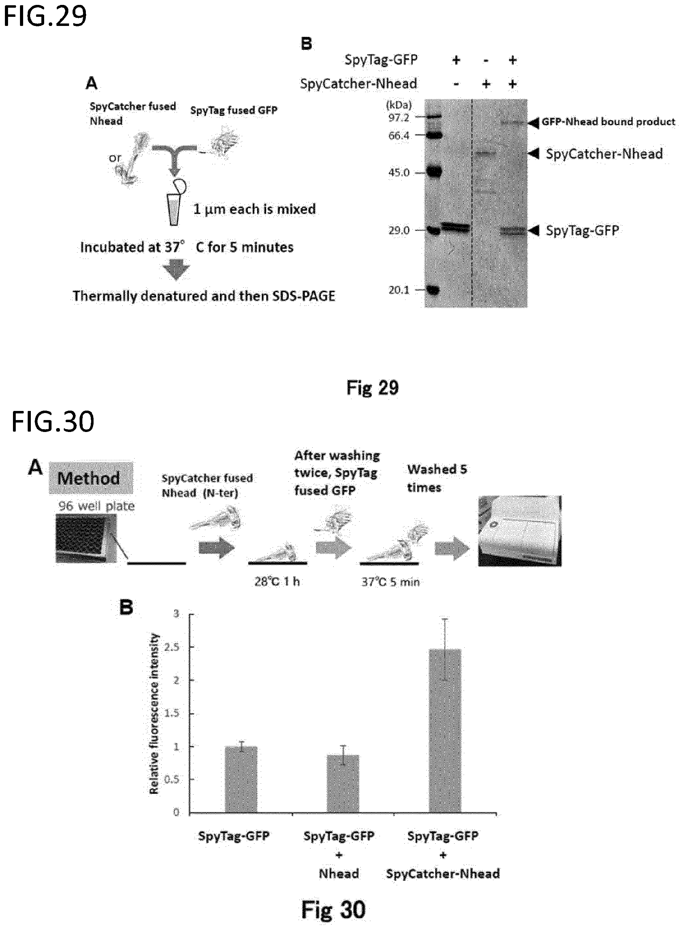

[Figure 29] Figure **29** shows that SpyCatcher-Nhead binds to SpyTag-GFP. Figure **29A** shows an experimental system for detecting binding of SpyCatcher-Nhead to SpyTag-GFP. Figure **29B** shows results of detecting binding of SpyCatcher-Nhead to SpyTag-GFP by CBB staining. In Figure **29B****,** CBB staining data corresponds to, in order from the left, molecular weight marker, SpyTag-GFP alone, SpyCatcher-Nhead alone, and mixture of SpyCatcher-Nhead and SpyTag-GFP. The left side shows the molecular weight, and the arrows on the right side indicate the molecular weights of GFP-Nhead binding product, SpyCatcher-Nhead fusion peptide, and SpyTag-GFP fusion peptide.
[Figure 30] Figure **30** shows that SpyTag-GFP can be immobilized onto a polystyrene plate via SpyCatcher-Nhead. Figure **30A** shows a model diagram for immobilizing SpyTag-GFP by immobilizing SpyCatcher-Nhead onto a polystyrene (PS) plate and binding SpyTag-GFP thereto. Figure **30B** shows results of measuring the amount of immobilization of GFP using fluorescence intensity of GFP. The graph shows results of GFP molecule immobilization test from coating a polystyrene plate using, in order from the left, SpyTag-GFP alone, SpyTag-GFP and Nhead, and SpyTag-GFP and SpyCatcher-Nhead. The vertical axis of the graph indicates the relative fluorescence intensity (excitation wavelength of 485 nm/fluorescence wavelength of 535 nm).

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical concepts that are particularly used herein are described hereinafter when appropriate.

### (Definitions of Terms)

As used herein, "adhere, adhesion" and "immobilize, immobilization" are used interchangeably and used in the sense commonly used in the art, and mean to make at least a portion of the subject of "adhesion" or "immobilization" at least relatively immobile from a certain position on the subject to be adhered or immobilized.

As used herein, "aggregate, aggregation" is used in the sense commonly used in the art and means that polypeptides or the like, in their dispersed state, gather in a mass with the polypeptides themselves, with other polypeptides or the like. Particularly when the same type of polypeptide chains aggregate together, it is referred to as self-aggregate, self-aggregation. For example, as to AtaA, the aggregation of multiple AtaA causes the phenomenon of forming a mass; thus, AtaA can be considered as having self-aggregating ability. Since the adhesive polypeptides of the present disclosure do not have a self-aggregation property, the polypeptides would not adhere in layers in one example. Thus, it is advantageous in the situation where the laminated adhesion is not preferable.

By lacking a self-aggregation property, polypeptides in an adhesive composition can disperse homogeneously to produce a state with homogeneous stress, so that the composition can more firmly adhere or immobilize.

In addition, a property of not self-aggregating while maintaining adhesion is useful in the following applications. Specifically, the adhesive polypeptide of the present disclosure is useful in controlling a function of a fused product and functionality-imparting entity in a separation process in pharmaceutical/chemical product industries, regenerative medicine, cell engineering, medical engineering, biosensors, and material engineering such as surface modification and designing of functional materials by causing adhesion or immobilization of the fused product or functionality-imparting entity in a single layer.

As used herein, "bind" and "bound" are used in the sense commonly used in the art and means a physical or chemical association between two substances, or a combination thereof. In particular, in the present disclosure, the interaction between adhesive polypeptide and streptavidin, neutral avidin, variants thereof, or the like falls under the "bind" herein. As used herein, "binding" is also referred to as "specific binding" because it corresponds to a specific interaction. On the other hand, adhesion or immobilization is due to non-specific interactions.

As used herein, "separation" and "separate" are used in the sense commonly used in the art and means that in two or more substances that are in a "bound" state, at least a part of the "bound" state between the substances is released.

As used herein, "disintegration" and "disintegrate" are used in the sense commonly used in the art and means that in two or more substances in an "aggregated" state, at least a part of the "aggregated" state between the substances is released.

As used herein, "AtaA" is a protein belonging to the trimeric autotransporter adhesin (TAA) family possessed by Gram-negative bacteria. AtaA forms a homotrimer and has a domain group structure with a signal peptide-Nhead-Nstalk-Chead-Cstalk-Membrane anchor arranged therein in this order from the amino terminal to the carboxy terminal. In addition, Nhead, Nstalk, Chead, and Cstalk include various domains such as Ylhead, Trp-ring, GIN and FGG, and also include some domains repeatedly. Respective domains are linked by a structure such as a neck or a coiled coil. The amino acid sequence and nucleic acid sequence of the fragment sequence of AtaA that does not include a signal peptide at the N-terminal but is added with methionine at the beginning and the binding site for the outer membrane of a microorganism cell is deleted so that it is in a separated state from the cell are shown in SEQ ID NOs: 1 and 2, respectively. In SEQ ID NOs: 1 and 2, Nhead (including Neck) corresponds to amino acid positions 2 to 258 and nucleic acid positions 4 to 774, Nstalk corresponds to amino acid positions 259 to 2846 and nucleic acid positions 775 to 8538, Chead (not including Neck) corresponds to amino acid positions 2855 to 3093 and nucleic acid positions 8563 to 9279, and Cstalk (including β-barrel inclusions) corresponds to amino acid positions 3094 to 3518 and nucleic acid positions 9280 to 10554. In addition, in the Nhead domain, there are two loop structures that are related to adhesive property, where these loop structures correspond to amino acid positions 22-50 and 161-175, and nucleic acid positions 64-150 and 481-525, respectively. Chead can be associated with self-aggregation.

As used herein, "*Acinetobacter*" means the genus *Acinetobacter* in the taxonomy. The genus *Acinetobacter* typically includes the *Acinetobacter* sp. Tol5 strain. The *Acinetobacter* sp. Tol5 strain is a strain with toluene resolution ability, isolated from the exhaust gas treatment reactor and is available from Katsutoshi Hori Laboratory, Nagoya University. The adhesive peptide of the present disclosure include, but are not limited to, those produced with the *Acinetobacter* sp. Tol5 strain belonging to the genus *Acinetobacter.*

As used herein, the "functionality-imparting entity" is used in the sense commonly used in the art and means those that have any functions or actions. The functionality-imparting entity is bound by the adhesive polypeptide or fragment thereof in the present disclosure. The functions or actions are typically various functions or actions useful in a separation process in pharmaceutical/chemical product industries, regenerative medicine, cell engineering, medical engineering, biosensors, material engineering such as surface modification and designing of functional materials and the like, and include, but are not limited to, functions such as labeling. In one embodiment, the functionality-imparting entity is a protein and/or peptide. In a specific embodiment, the functionality-imparting entity is streptavidin, neutral avidin or a variant thereof. In another embodiment, a functionality-imparting entity is a cell.

As used herein, "streptavidin" is used in the sense commonly used in the art and means streptavidin produced by the genus Streptomyces, or variants, derivatives or equivalents thereof. Examples of the amino acid sequence thereof include, but are not limited to, P22629-1, and examples of the nucleic acid sequence thereof include, but are not limited to, those specified by X03591. The "streptavidin" herein typically binds to biotin with high affinity. As used herein, "neutral avidin" is used interchangeably with "NeutrAvidin" and the like in the same sense, and means a variant of avidin (particularly, a sugar chain-removed substance) which has a pI value near neutrality and in which non-specific binding is suppressed. "Neutral avidin" also typically binds to biotin with high affinity. "Streptavidin" and "neutral avidin" have the property of specifically binding to the adhesive polypeptide of the present disclosure.

As used herein, "liposome" is used in a meaning that is commonly used in the art, referring to a spherical self-aggregated entity formed by amphiphilic molecules including a polar hydrophilic group and a lipophilic lipid group, which is a vesicle forming a substantially closed structure in an aqueous medium and having a lipid bilayer structure. A liposome can include a DNA, protein, peptide, or the like inside. In a specific embodiment, a liposome is used as a transport vesicle. In another embodiment, a liposome is useful as an artificial cell.

As used herein, a "corresponding" amino acid or nucleic acid or moiety refer to: an amino acid or nucleotide that has or is expected to have a similar action as a given amino acid or nucleotide or moiety in the polypeptide or polynucleotide to be used as a reference for comparison, in a certain polypeptide or polynucleotide molecule (e.g., AtaA and the like); and for adhesive polypeptides in particular, an amino acid that is present at similar positions in the site which is essential for adhesion and makes a similar contribution to adhesive property. For example, as for an antisense molecule, it can be a similar part in the ortholog corresponding to a particular part of the antisense molecule. The corresponding amino acid can be, for example, a specific amino acid that is cysteinylated, glutathioneized, S-S bond formed, oxidized (e.g., methionine side chain oxidation), formylated, acetylated, phosphorylated, glycosylated, myristilized, and the like. Alternatively, the corresponding amino acid can be the amino acid responsible for trimerization. Such a "corresponding" amino acid or nucleic acid may be a region or domain over a certain range. Therefore, in such cases, they are referred to herein as a "corresponding" region or domain.

As used herein, "fusion" means that two or more molecules (e.g., polypeptides or nucleic acids) or parts thereof are linked by covalent binding. When two or more moieties are polypeptides and they form a covalently bound complex, they are fusions or fusion products produced by "fusion", or fusion proteins or fusion peptides, which can also be referred to as chimeric peptides. Regularly, in the case of a polypeptide fusion, a fusion of nucleic acids is prepared by linking the base sequences of nucleic acids encoding two types of polypeptides, and the fusion is used as a design drawing to produce a fusion peptide as a recombinant protein.

As used herein, "complex" is used in the sense commonly used in the art and means any construct including two or more moieties. For example, if one of the moieties is a polypeptide, the other of the moieties may be a polypeptide or any other substances (e.g., sugars, lipids, nucleic acids, other hydrocarbons, cells, etc.). Two or more moieties that constitute the complex herein can be bound in a non-covalent manner (e.g., hydrogen bond, ionic bond, hydrophobic interaction, van der Waals force, and the like). Therefore, as used herein, the "complex" includes molecules formed by linking a plurality of types of molecules, such as polypeptides, polynucleotides, lipids, sugars, and small molecules. A complex formed through a bond other than a covalent bond can be separately into two or more portions by a simple method. Examples of the simple method include operations such as application of a shear stress by physical means such as stirring, addition of a compound, changing solution conditions such as pH or salt concentration, and changing the temperature.

It is well known in the art that an amino acid can be substituted with another amino acid having the same hydrophobicity index to still produce a protein with the same biological function (e.g., protein with equivalent adhesive property). In such an amino acid substitution, hydrophobicity index is preferably within ± 2, more preferably within ± 1, and still more preferably within ± 0.5. It is understood in the art that such an amino acid substitution based on hydrophobicity is efficient. A hydrophilicity index is also considered for the preparation of variants. As described in US Patent No. 4,554,101, the following hydrophilicity indices are assigned to amino acid residues: arginine (+3.0); lysine (+3.0); aspartic acid (+3.0 ± 1); glutamic acid (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid can be substituted with another amino that has the same hydrophilicity index and can still provide a biological equivalent. In such an amino acid substitution, the hydrophilicity index is preferably within ± 2, more preferably within ± 1, and still more preferably within ± 0.5.

In the present disclosure, "conservative substitution" means a substitution in which the hydrophilicity index and/or the hydrophobicity index of the substituting amino acid is similar to that of the original amino acid as described above. Examples of the conservative substitution are well known to those of skill in the art and include, but are not limited to, for example, substitutions within each of the following groups: arginine and lysine; glutamic acid and aspartic acid; serine and threonine; glutamine and asparagine; and valine, leucine, isoleucine, and the like.

The "variant", "derivative", "analog" or "mutant" (of adhesive polypeptides and the like) as used herein are used interchangeably, and the above terms preferably include molecules including regions that are substantially homologous to the protein (e.g., adhesive polypeptide) of interest, although not intended to be limiting; and in the case of amino acid sequence, nucleic acid sequence (nucleotide sequence, base sequence), such sequences are referred to as "altered sequence", "derived sequence", "analogous sequence", and "mutated sequence". Such molecules, in various embodiments, are at least 30%, 40%, 50%, 60%, 70%, 80%, 90%, 95%, or 99% identical, throughout amino acid sequences of the same size, or in comparison with sequences to be aligned by conducting alignment with a computer homology program that is known in the art; or nucleic acids encoding such molecules can hybridize to sequences encoding component proteins under (highly) stringent, moderately stringent, or non-stringent conditions. This means that each is a product of protein alteration by amino acid substitutions, deletions and additions, in which the variant or derivative thereof still exhibits the biological function of the original protein, but not necessarily to the same degree. The variant may have a sequence that is preferably 70% or more identical, more preferably 80% or more identical, even more preferably 90% or more identical, still even more preferably 95% or more identical, 98% or more identical, 99% or more identical, to the original sequence. For example, it is also possible to investigate the biological function of such a protein by appropriate and available *in vitro* assays described herein or known in the art. "Functionally active" as used herein refers to a polypeptide, i.e., a fragment or derivative, having a structural, regulatory, or biochemical function of protein, such as biological activity, according to aspects to which the polypeptide, i.e., the fragment or derivative, of the present disclosure is associated herein. Accordingly, in one embodiment, the variant of the present disclosure refers to a functional equivalent.

As used herein, a fragment of an adhesive polypeptide is a polypeptide comprising any region of the adhesive polypeptide, which does not necessarily need to have all of the biological functions of a naturally-occurring adhesive polypeptide, as long as it function as intended in the present disclosure (e.g., immobilize and/or adhere without self-aggregation).

As used herein, "protein", "polypeptide", "oligopeptide", and "peptide" are used with the same meaning herein and refer to a polymer of amino acids of any length. The length of the protein, polypeptide, or peptide of the present disclosure is preferably at least 237 amino acid residues and at most 3364 amino acid residues, and more preferably at least 256 amino acid residues and at most 2846 amino acid residues. The polymer may be linear, branched or cyclic. The amino acids may be natural or non-natural, or may be altered amino acids. The term may also include those assembled into a complex of multiple polypeptide chains. The term also includes naturally or artificially altered amino acid polymers. Such alterations include, for example, disulfide bond formation, glycosylation, lipidation, acetylation, phosphorylation, methylation, trimethylation or any other manipulation or alteration (e.g., conjugation with a labeling component) This definition also includes, for example, polypeptides including one or more analogs of amino acids (including, for example, unnatural amino acids), peptide-like compounds (e.g., peptoids) and other alterations known in the art. As used herein, "amino acid" is a general term for organic compounds having an amino group and a carboxyl group. When the polypeptide according to the embodiments of the present disclosure includes a "specific amino acid sequence", any amino acid in the amino acid sequence may be chemically modified. In addition, any amino acid in the amino acid sequence may form a salt or a solvate. Furthermore, any amino acid in the amino acid sequence may be L-type or D-type. Even in such cases, the protein according to the embodiments of the present disclosure can be said to include the above "specific amino acid sequence". As to the chemical modification that an amino acid included in a protein undergoes *in vivo,* knowns are, for example, N-terminal modification (e.g., acetylation, myristoylation, etc.), C-terminal modification (e.g., amidation, glycosylphosphatidylinositol addition, etc.), side chain modification (e.g., phosphorylation, sugar chain addition, etc.) or the like. The amino acids may be any natural or non-natural amino acids that satisfy the objectives of the present disclosure.

As used herein, "polynucleotide", "oligonucleotide" and "nucleic acid" are used with the same meaning herein and refer to a polymer of nucleotide of any length. The term also includes an "oligonucleotide derivative" or "polynucleotide derivative". The "oligonucleotide derivative" or "polynucleotide derivative" refers to an oligonucleotide or polynucleotide that includes a derivative of a nucleotide or has an unusual bond between nucleotides and is used interchangeably. Specific examples of such oligonucleotides include, for example 2'-O-methyl-ribonucleotide, an oligonucleotide derivative in which the phosphate diester bond in the oligonucleotide is converted to a phosphorothioate bond, an oligonucleotide derivative in which the phosphate diester bond in the oligonucleotide is converted to an N3'-P5'phosphodiester bond, an oligonucleotide derivative in which a ribose and a phosphate diester bond in the oligonucleotide are converted into a peptide nucleic acid bond, an oligonucleotide derivative in which uracil in the oligonucleotide is substituted by C-5 propynyl uracil, an oligonucleotide derivative in which uracil in the oligonucleotide is substituted by C-5 thiazole uracil, an oligonucleotide derivative in which cytosine in the oligonucleotide is substituted by C-5 propynylcytosine, an oligonucleotide derivative in which cytosine in the oligonucleotide is substituted by phenoxazine-modified cytosine, an oligonucleotide derivative in which ribose in DNA is substituted by 2'-O-propylribose, and an oligonucleotide derivative in which ribose in the oligonucleotide is substituted by 2'-methoxyethoxyribose. Unless otherwise indicated, the specific nucleic acid sequence is also intended to include sequences that are explicitly shown, as well as the conservatively altered variants (e.g., a degenerate codon substitution) and complementary sequences thereof. Specifically, the degenerate codon substitution can be achieved by creating a sequence in which the third position of one or more selected (or all) codons is substituted by a mixed base and/or deoxyinosine residue (Batzer et al., Nucleic Acid Res.19:5081(1991); Ohtsuka et al., J. Biol. Chem. 260: 2605-2608(1985); Rossolini et al., Mol. Cell. Probes 8:91-98(1994)). As used herein, "nucleic acid" is also used interchangeably with genes, cDNAs, mRNAs, oligonucleotides, and polynucleotides. As used herein, "nucleotide" may be natural or non-natural.

As used herein, "gene" means a factor that defines a genetic trait, and "gene" may also refer to "polynucleotide", "oligonucleotide" and "nucleic acid".

As used herein, "homology" of a gene means the degree of identity of two or more gene sequences to each other, and generally, having "homology" means a high degree of identity or similarity. Thus, as the homology of two certain genes increases, the identity or similarity of the sequences thereof increases. Whether or not two types of genes are homologous can be determined by direct comparison of the sequences, or in the case of nucleic acids, can be examined by hybridization methods under stringent conditions. In direct comparison of two gene sequences, the genes thereof are homologous if the DNA sequences are typically at least 50% identical, preferably at least 70% identical, more preferably at least 80%, 90%, 95%, 96%, 97%, 98% or 99% identical, between the gene sequences. Similarly, the concept of "homology" also applies to amino acid sequences, in which case the numerical values of identity etc. can be interpreted by replacing them with amino acid sequences. Accordingly, as used herein, "homolog" or "homologous gene product" means a protein in another species, preferably a microorganism, more preferably a bacterium, which exerts the same biological function as the protein component of the fusion or the like to be further described herein. Such a homolog is also sometimes referred to as an "ortholog gene product". It is understood that such a homolog, homologous gene product, ortholog gene product, etc. can also be used as long as they meet the objectives of the present disclosure.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.7.1 (published on October 19, 2017) of the NCBI. Herein, values for "identity" generally refer to a value obtained when aligned under the default conditions using BLAST. However, when a higher value is obtained by changing a parameter, the highest value is considered the value of identity. When identity is evaluated in a plurality of regions, the highest value thereamong is considered the value of identity. "Similarity" is a value calculated by taking into consideration a similar amino acid in addition to identity.

In one embodiment of the present disclosure, the "several" may be, for example, 10, 8, 6, 5, 4, 3, or 2, or less than or equal to any of these values. It is known that a polypeptide with one or several amino acid residue deletions, additions, insertions or substitutions with other amino acids, still maintains its biological activity (Mark et al., Proc Natl Acad Sci USA.1984 Sep; 81(18): 5662-5666., Zoller et al., Nucleic Acids Res. 1982 Oct 25; 10(20): 6487-6500., Wang et al., Science.1984 Jun 29; 224(4656): 1431-1433.). Peptides with deletions and the like can be prepared, for example, by a site-specific mutagenesis method, a random mutagenesis method, or the like. As a site-specific mutagenesis method, for example, KOD-Plus-Mutagenesis Kit (TOYOBO CO., LTD.) can be used. It is possible to select a polypeptide having the same activity as the wild-type from the mutant-type polypeptides in which a deletion or the like has been introduced, by performing various characterizations such as FACS analysis and ELISA.

In one embodiment of the present disclosure, the numerical value of identity or the like, i.e., "70% or greater", can be, for example, 70% or greater, 75% or greater, 80% or greater, 85% or greater, 90% or greater, 95% or greater, 96% or greater, 97% or greater, 98% or greater, 99% or greater, or 100% or greater, or within a range between any two of numerical values of such starting points. The above "identity" is computed by computing the ratio of the number of amino acids homologous in two or more amino acid sequences in accordance with a known method described above. Specifically, amino acid sequences in a group of amino acid sequences to be compared are aligned before computing the ratio, and a space is introduced in a part of the amino acid sequences if needed to maximize the ratio of identical amino acids. A method for alignment, ratio computation method, comparison method, and computer program associated therewith are conventional and well known in the art (e.g., aforementioned BLAST or the like). As used herein, "identity" and "similarity" can be represented by a value measured by NCBI's BLAST, unless specifically noted otherwise. Blastp can be used with the default settings as the algorithm for comparing amino acid or base sequences with BLAST. Measurement results are quantified as Positives or Identities.

As used herein, a "polynucleotide that hybridizes under stringent conditions" refers to well-known conditions commonly used in the art. Such a polynucleotide can be obtained by using a polynucleotide selected from the polynucleotides of the present disclosure as a probe and using a colony hybridization method, a plaque hybridization method or a southern blot hybridization method. Specifically, the above means such a polynucleotide that can be identified by performing hybridization at 65°C in the presence of 0.7 to 1.0 M NaCl using a filter with immobilized DNA from a colony or plaque, followed by washing the filter under 65°C conditions with a 0.1 to 2-fold concentration SSC (saline-sodium citrate) solution (where the composition of the 1-fold concentration SSC solution is 150 mM sodium chloride and 15 mM sodium citrate). For the "stringent conditions", the conditions below, for example, can be adopted: (1) use of low ionic strength and high temperature for washing (e.g., at 50°C, 0.015M sodium chloride/0.0015M sodium citrate/0.1% sodium dodecyl sulfate); (2) use of a denaturing agent such as formamide during hybridization (e.g., at 42°C, 50% (v/v) formamide and 0.1% bovine serum albumin/0.1% ficoll/0.1% polyvinylpyrrolidone/50 mM and pH 6.5 sodium phosphate buffer, and 750 mM sodium chloride, 75 mM citrate sodium), or (3) incubation overnight at 37°C in a solution containing 20% formamide, 5 × SSC, 50 mM sodium phosphate (pH 7.6), 5 × denhard solution, 10% dextran sulfate, and 20 mg/ml denatured sheared salmon sperm DNA, followed by washing the filter with 1 × SSC at about 37 to 50°C. Note that the formamide concentration may be 50% or higher. Washing time may be 5, 15, 30, 60, or 120 minutes, or longer. Multiple factors such as temperature and salt concentration can be considered as factors that affect the stringency of the hybridization reaction, and for details, Ausubel et al., Current Protocols in Molecular Biology, Wiley Interscience Publishers,(1995) may be referenced. Examples of "highly stringent conditions" are 0.0015M sodium chloride, 0.0015M sodium citrate, 65 to 68°C, or 0.015M sodium chloride, 0.0015M sodium citrate, and 50% sodium citrate, at 42°C. Hybridization can be performed in accordance with the method described in experimental publications such as Molecular Cloning 2nd ed., Current Protocols in Molecular Biology, Supplement 1-38, DNA Cloning 1: Core Techniques, A Practical Approach, Second Edition, Oxford University Press (1995). In this regard, sequences containing only the A sequence or only the T sequence are preferably excluded from the sequences to be hybridized under stringent conditions. Moderate stringent conditions can be easily determined by one of ordinary skill in the art on the basis of, for example, the length of the DNA, shown in Sambrook et al., Molecular Cloning: A Laboratory Manual, No. 3, Vol.1, 7. 42-7. 45 Cold Spring Harbor Laboratory Press, 2001; and as to nitrocellulose filters, included are the use of: hybridization conditions with a pre-cleaning solution of 5 × SSC, 0.5% SDS, and 1.0 mM EDTA (pH 8.0), and at about 40 to 50°C, about 50% formamide, 2 × SSC-6 × SSC (or other similar hybridization solution, such as Stark's solution, in about 50% formamide at about 42°C); and washing conditions of about 60°C, 0.5 × SSC, 0.1% SDS. Accordingly, the peptides used in this disclosure also include polypeptides coded by nucleic acid molecules that hybridize under highly or moderately stringent conditions to nucleic acid molecules that code the polypeptides specifically described in the present disclosure.

The adhesive polypeptides of the present disclosure may preferably be "purified" or "isolated". A "purified" substance or biological factor (e.g., nucleic acid or protein) as used herein refers to one in which at least some of the factors naturally associated with the substance or biological factor have been removed. Thus, the purity of the biological factor in the purified biological factor is usually higher (i.e., enriched) than the purity of the biological factor in the state in which the biological factor is normally present. The term, "purified", as used herein means that there is preferably at least 75% by weight, more preferably at least 85% by weight, even more preferably at least 95% by weight, and most preferably at least 98% by weight, of the same type of biological factor present. The substance or biological factor used in the present disclosure is preferably a "purified" substance. An "isolated" substance or biological factor (e.g., nucleic acid or protein) as used herein refers to one in which the factors naturally associated with the substance or biological factor have been substantially removed. The term, "isolated", as used herein does not necessarily have to be expressed in purity as it varies in accordance with the objective thereof, but if necessary, means that there is preferably at least 75% by weight, more preferably at least 85% by weight, even more preferably at least 95% by weight, and most preferably at least 98% by weight, of the same type of biological factor present. The substance used in the present disclosure is preferably an "isolated" substance or biological factor.

As used herein, "fragment" refers to a polypeptide or polynucleotide with a sequence length of 1 to n-1 with respect to a full-length polypeptide or polynucleotide (with the length of n). The length of the fragment can be varied as appropriate according to the objective thereof, and the lower limit of the length thereof, in the case of polypeptides, includes, for example, 3, 4, 5, 6, 7, 8, 9, 10, 15, 20, 25, 30, 40, 50 and more amino acids; and lengths represented by integers not specifically listed here (e.g., 11) can also be appropriate as lower limits. A fragment of the polypeptide of the present disclosure consists of preferably 237 or more and 3364 or less amino acids and more preferably 256 or more and 2846 or less amino acids. Examples of the lower limit include, but are not limited to, 237 amino acids, 256 amino acids, 300 amino acids, 400 amino acids, 500 amino acids, 600 amino acids, 700 amino acids, 800 amino acids, 900 amino acids, 1000 amino acids, and the like. Examples of the upper limit include, but are not limited to, 1000 amino acids, 1100 amino acids, 1200 amino acids, 1300 amino acids, 1400 amino acids, 1500 amino acids, 1600 amino acids, 1700 amino acids, 1800 amino acids, 1900 amino acids, 2000 amino acids, 2100 amino acids, 2200 amino acids, 2300 amino acids, 2400 amino acids, 2500 amino acids, 2600 amino acids, 2700 amino acids, 2800 amino acids, 2846 amino acids, 2900 amino acids, 3000 amino acids, 3100 amino acids, 3200 amino acids, 3300 amino acids, and 3364 amino acids. The length can be any other length, as long as a desired property (e.g., lack of self-aggregation property or maintaining an adhesive property) is maintained. Further, in the case of polynucleotides, included are 5, 6, 7, 8, 9, 10, 15, 20, 25, 30, 40, 50, 75, 100 and more nucleotides; and lengths represented by integers not specifically listed here (e.g., 11) can also be appropriate as lower limits. As used herein, it is understood that such a fragment falls within the scope of the present disclosure, for example, if the full length one functions as an adhesive molecule, as long as the fragment itself also functions as an adhesive polypeptide.

As used herein, "biological function" refers to a specific function that a certain gene or a nucleic acid molecule or polypeptide associated therewith may have *in vivo* or *in vitro* in the context of the gene, nucleic acid molecule or polypeptide, the examples of which include, but are not limited to, for example, an adhesive property and the like. As used herein, the biological function can be exerted by the corresponding "biological activity". As used herein, "biological activity" refers to the activity that a factor (e.g., polynucleotide, protein, etc.) may have, and includes activities that exert various functions (e.g., an adhesive property). The "biological activity" may be an activity exerted *in vivo* or an activity exerted *in vitro* by secretion or the like. For example, if a certain factor is an enzyme, the biological activity thereof includes the enzyme activity thereof. Such biological activity can be measured by techniques well known in the art. Thus, "activity" refers to various measurable indicators that indicate or reveal binding (either direct or indirect) or various measurable indicators that affect the response (i.e., have a measurable effect in response to some exposure or stimulus), which may also include, for example, affinities for compounds that directly bind to the polypeptides or polynucleotides of the present disclosure, or for example, the amount of upstream or downstream protein after some stimulus or event, or other similar scales of function.

As used herein, "expression" of a gene, polynucleotide, polypeptide or the like refers to the gene or the like to undergo a certain action *in vivo* thus turning into a different form. This preferably refers to a gene, a polynucleotide or the like to be transcribed and translated into the form of a polypeptide; however, it is also an aspect of the expression that they are transcribed to produce mRNA. Accordingly, the "expression product" as used herein includes such a polypeptide or protein, or mRNA. More preferably, the form of such a polypeptide may be the one that is post-translationally processed. For example, the expression level of an adhesive polypeptide can be determined by any method. Specifically, assessing the amount of mRNA of the adhesive polypeptide, the amount of the adhesive polypeptide, and the biological activity of the adhesive polypeptide allows finding out of the expression level of the adhesive polypeptide. The amount of mRNA of the adhesive polypeptide, polypeptide or protein can be determined by methods as detailed elsewhere herein or by other methods known in the art.

As used herein, "functional equivalent" means any entity that has the same objective function but a different structure with respect to the original entity of interest. Thus, it is understood that the functional equivalent of the "adhesive polypeptide" of the present disclosure is not the adhesive polypeptide of the present disclosure itself, but includes a mutant or variant thereof (e.g., an amino acid sequence variant or the like), which has a biological action of the adhesive polypeptide as well as such a functional equivalent (including, for example, a nucleic acid encoding the mutant or variant, a vector including the nucleic acid, a cell, and the like) which, at the time of action, can be transformed into a mutant or variant that has the biological action of the adhesive polypeptide. In the present disclosure, it is understood that the functional equivalent of the adhesive polypeptide can be used in the same manner as the adhesive polypeptide, without special mention. Functional equivalents can be found by searching databases and the like. As used herein, "searching" refers to finding another nucleic acid base sequence with a particular function and/or property by utilizing a certain nucleic acid base sequence electronically, biologically or otherwise. Examples of electronic searching include, but are not limited to, BLAST (Altschul et al., J. Mol. Biol. 215: 403-410(1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)). Examples of biological searching include, but are not limited to, stringent hybridization, microarrays with genomic DNA attached to nylon membranes, etc. or microarrays attached to glass plates (microarray assays), PCR and *in situ* hybridization. Herein, it is intended that the genes used in the present disclosure should also include the corresponding genes identified by such electronic and biological searching.

As for the functional equivalent of the present disclosure (polypeptide, etc.), it is possible to use amino acid sequences in which one or more amino acids are inserted, substituted or deleted, or added to one or both ends. As used herein, "one or more amino acids are inserted, substituted or deleted, or added to one or both ends" means that alterations have been made with the substituting of a plurality of amino acids that may occur naturally, by well-known technical methods such as site-specific mutagenesis methods, or by natural mutations. An altered amino acid sequence can have, for example, 1 to 30, preferably 1 to 20, more preferably 1 to 9, still more preferably 1 to 5, and particularly preferably 1 to 2 amino acid insertions, substitutions, or deletions, or additions to one or both ends thereof. The altered amino acid sequence may be preferably such an amino acid sequence that has one or more (preferably one or several, or 1, 2, 3, or 4) conservative substitutions in the amino acid sequence of SEQ ID NO: 1 or 4. "Conservative substitution" herein means a substitution of one or more amino acid residues with other chemically similar amino acid residues so as not to substantially alter the function of the protein. Examples thereof include substitutions of a hydrophobic residue with another hydrophobic residue, substitutions of a polar residue with another polar residue having the same charge, and the like. Functionally similar amino acids that can be substituted in this manner are known in the art for each amino acid. Specific examples include alanine, valine, isoleucine, leucine, proline, tryptophan, phenylalanine, methionine, and the like for nonpolar (hydrophobic) amino acids, and glycine, serine, threonine, tyrosine, glutamine, asparagine, cysteine, and the like for polar (neutral) amino acids. Examples of positively charged (basic) amino acids include arginine, histidine, lysine, and the like. Further, examples of a negatively-charged (acidic) amino acid include aspartic acid, glutamic acid, and the like.

As used herein, "support" refers to a substance capable of carrying a peptide, protein, polynucleotide, cell, bacterium, or virus and a sugar, lipid, or other low molecules. The material for use as a support can be any material that can form a solid surface, and examples thereof include, but are not limited to, a lipid double structure (e.g., liposome), glass, silica, silicon, ceramic, silicon dioxide, plastic, metal (including alloy), titanium, acrylic material, natural and synthetic polymer (e.g., polystyrene, polypropylene, polyethylene, polyvinyl chloride, cellulose, chitosan, dextran, nylon, polyurethane, polycarbonate, polylactic acid, and polytetrafluoroethylene (PTFE)).

As used herein, "kit" refers to a unit providing parts to be provided (e.g., an enzyme, buffer, user manual, and the like) which are generally separated into two or more segments. Such a kit form is preferred when providing a composition, which should not be provided in a mixed state for stability or the like and is preferably used by mixing immediately prior to use. Such a kit advantageously comprises an instruction or user manual describing how the provided parts (e.g., enzymes) are used or how a reagent or waste fluid after use should be processed. When a kit is used as a reagent kit herein, the kit generally comprises an instruction or the like describing the method of use of the enzyme, or the like.

As used herein, "instruction" is a document with an explanation of the method of use of the present disclosure for users. The instruction provides description for instructing the method of use of the present disclosure. If required, the instruction is prepared in accordance with a format specified by a regulatory authority of the country in which the present invention is practiced (e.g., Ministry of Health, Labour and Welfare, Ministry of Agriculture, Forestry and Fisheries, or the like in Japan, Food and Drug Administration (FDA) or Department of Agriculture (USDA) in the U.S., or the like), with an explicit description showing approval by the regulatory authority. An instruction can be provided in, but not limited to, paper media. An instruction can also be provided in a form such as electronic media (e.g., web sites provided on the Internet or emails).

### (Preferred embodiments)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

### (Adhesive polypeptide without a self-aggregation property)

In one aspect, the present disclosure provides a novel fragment of an adhesive polypeptide or a variant thereof, which does not have a self-aggregation property and has an adhesive property, and a nucleic acid encoding the same.

In a representative aspect, the present disclosure provides a fragment of AtaA or a variant thereof, which does not have a self-aggregation property and has an adhesive property.

In a certain embodiment, the present disclosure typically provides a representative sequence of an adhesive polypeptide obtained from an *Acinetobacter* sp. Tol5 strain, or a variant thereof.

In one embodiment, the present disclosure provides a polypeptide comprising a fragment of the amino acid sequence set forth in SEQ ID NO: 1 or a variant thereof, wherein the polypeptide does not have a self-aggregation property and has an adhesive property.

In a specific embodiment, the adhesive polypeptide of the present disclosure comprises a sequence corresponding to amino acid positions 22 to 50 and 161 to 175 of the amino acid sequence set forth in SEQ ID NO: 1 or a variant sequence thereof, and one or more amino acid substitutions, additions, deletions, or combinations thereof in a range of a sequence corresponding to amino acid positions 2847 to 3093 of the amino acid sequence set forth in SEQ ID NO: 1. Preferably, the range of a sequence corresponding to amino acid positions 2847 to 3093 of the amino acid sequence set forth in SEQ ID NO: 1 is a range corresponding to amino acid positions 2855 to 3093 of the amino acid sequence set forth in SEQ ID NO: 1. More preferably, the adhesive polypeptide of the present disclosure has a deletion of the entire sequence corresponding to amino acid positions 2855 to 3093 of the amino acid sequence set forth in SEQ ID NO: 1. In a specific embodiment, the variable sequence comprises an alteration by a conservative substitution.

In a specific embodiment, the present disclosure provides a polypeptide, which is
(1) a polypeptide comprising, in a sequence corresponding to amino acid positions 1 to 3518 of the amino acid sequence set forth in SEQ ID NO: 1 or a variant sequence thereof or a fragment thereof,
   at least one or more amino acid substitutions, additions, deletions, or combinations thereof in a sequence corresponding to amino acid positions 2855 to 3093 of SEQ ID NO: 1, and
   a sequence having a sequence corresponding to amino acid positions 22 to 50 and 161 to 175 of SEQ ID NO: 1 or a variant sequence thereof,
(2) a polypeptide comprising, in a sequence corresponding to amino acid positions 1 to 3093 of the amino acid sequence set forth in SEQ ID NO: 1 or a variant sequence thereof or a fragment thereof,
   at least one or more amino acid substitutions, additions, deletions, or combinations thereof in a sequence corresponding to amino acid positions 2855 to 3093, and
   a sequence having a sequence corresponding to amino acid positions 22 to 50 and 161 to 175 or a variant sequence thereof,
(3) a polypeptide, which is a sequence corresponding to amino acid positions 1 to 2846 of the amino acid sequence set forth in SEQ ID NO: 1 or a variant sequence thereof or a fragment thereof, comprising
   a sequence corresponding to amino acid positions 22 to 50 and 161 to 175 or a variant sequence thereof,
(4) a polypeptide comprising a part of amino acid positions 1 to 3518 of the amino acid sequence set forth in SEQ ID NO: 1 (wherein "a part" refers to a sequence with a deleted portion in a full length sequence, encompassing fragments as well as sequences with an intermediate portion knocked out),

at least one or more amino acid substitutions, additions, deletions, or combinations thereof in a sequence corresponding to amino acid positions 2855 to 3093 of SEQ ID NO: 1, and
a sequence corresponding to amino acid positions 22 to 50 and 161 to 175 of SEQ ID NO: 1 or a variant sequence thereof,

(5) a polypeptide, which is a sequence corresponding to amino acid positions 1 to 2854 of the amino acid sequence set forth in SEQ ID NO: 1 or a variant sequence thereof or a fragment thereof, having
a sequence corresponding to amino acid positions 22 to 50 and 161 to 175 or a variant sequence thereof,

(6) a polypeptide, which is a sequence corresponding to amino acid positions 1 to 268 of the amino acid sequence set forth in SEQ ID NO: 1 or a variant sequence thereof or a fragment thereof, having
a sequence corresponding to amino acid positions 22 to 50 and 161 to 175 or a variant sequence thereof, or

(7) a polypeptide, which is a sequence corresponding to amino acid positions 1 to 257 of the amino acid sequence set forth in SEQ ID NO: 1 or a variant sequence thereof or a fragment thereof, having
a sequence corresponding to amino acid positions 22 to 50 and 161 to 175 or a variant sequence thereof.

In a specific embodiment, the present disclosure provides a polypeptide, which is
(a) a polypeptide, which is an amino acid sequence corresponding to amino acid positions 1 to 2854 of the amino acid sequence set forth in SEQ ID NO: 1 or a variant sequence thereof or a fragment thereof, having a sequence corresponding to amino acid positions 22 to 50 and 161 to 175 or a variant sequence thereof,
(b) a polypeptide, which is an amino acid sequence corresponding to amino acid positions 1 to 2846 of the amino acid sequence set forth in SEQ ID NO: 1 or a variant sequence thereof or a fragment thereof, having a sequence corresponding to amino acid positions 22 to 50 and 161 to 175 or a variant sequence thereof,
(c) a polypeptide, which is a sequence corresponding to amino acid positions 1 to 268 of the amino acid sequence set forth in SEQ ID NO: 1 or a variant sequence thereof or a fragment thereof, having a sequence corresponding to amino acid positions 22 to 50 and 161 to 175 or a variant sequence thereof, or
(d) a polypeptide, which is a sequence corresponding to amino acid positions 1 to 257 of the amino acid sequence set forth in SEQ ID NO: 1 or a variant sequence thereof or a fragment thereof, having a sequence corresponding to amino acid positions 22 to 50 and 161 to 175 or a variant sequence thereof.

In a specific embodiment, the present disclosure provides a polypeptide, which is
(1) a polypeptide, which is an amino acid sequence corresponding to amino acid positions 1 to 2854 of the amino acid sequence set forth in SEQ ID NO: 1 or a variant sequence thereof or a fragment thereof,
(2) a polypeptide, which is an amino acid sequence corresponding to amino acid positions 1 to 2846 of the amino acid sequence set forth in SEQ ID NO: 1 or a variant sequence thereof or a fragment thereof,
(3) a polypeptide, which is an amino acid sequence corresponding to amino acid positions 1 to 268 of the amino acid sequence set forth in SEQ ID NO: 1 or a variant sequence thereof or a fragment thereof, or
(4) a polypeptide, which is an amino acid sequence corresponding to amino acid positions 1 to 257 of the amino acid sequence set forth in SEQ ID NO: 1 or a variant sequence thereof or a fragment thereof.

In a specific embodiment, the adhesive polypeptide of the present disclosure is an amino acid sequence corresponding to amino acid positions 1 to 2854 or 2 to 2854 of the amino acid sequence set forth in SEQ ID NO: 1 or a variant sequence thereof or a fragment thereof. Preferably, the adhesive polypeptide of the present disclosure consists of an amino acid sequence corresponding to amino acid positions 1 to 2854 or 2 to 2854 of the amino acid sequence set forth in SEQ ID NO: 1.

In a specific embodiment, the adhesive polypeptide of the present disclosure is an amino acid sequence corresponding to amino acid positions 1 to 2846 or 2 to 2846 of the amino acid sequence set forth in SEQ ID NO: 1 or a variant sequence thereof or a fragment thereof. Preferably, the adhesive polypeptide of the present disclosure consists of an amino acid sequence corresponding to amino acid positions 1 to 2846 or 2 to 2846 of the amino acid sequence set forth in SEQ ID NO: 1.

In a specific embodiment, the adhesive polypeptide of the present disclosure is an amino acid sequence corresponding to amino acid positions 1 to 268 or 2 to 268 of the amino acid sequence set forth in SEQ ID NO: 1 or a variant sequence thereof or a fragment thereof. Preferably, the adhesive polypeptide of the present disclosure consists of an amino acid sequence corresponding to amino acid positions 1 to 268 or 2 to 268 of the amino acid sequence set forth in SEQ ID NO: 1.

In a specific embodiment, the adhesive polypeptide of the present disclosure is an amino acid sequence corresponding to amino acid positions 1 to 257 or 2 to 257 of the amino acid sequence set forth in SEQ ID NO: 1 or a variant sequence thereof or a fragment thereof. Preferably, the adhesive polypeptide of the present disclosure consists of an amino acid sequence corresponding to amino acid positions 1 to 257 or 2 to 257 of the amino acid sequence set forth in SEQ ID NO: 1.

In some embodiments, the adhesive polypeptide of the invention is derived from an *Acinetobacter* bacterium. In a specific embodiment, the *Acinetobacter* bacterium is of an *Acinetobacter* sp. Tol5 strain.

In some embodiments, the adhesive polypeptide of the present disclosure further comprises an amino acid sequence that is different from SEQ ID NO: 1. In a specific embodiment, the different amino acid sequence is fused with the adhesive polypeptide of the present disclosure. In one embodiment, the different amino acid sequence is an amino acid sequence encoding a SNAP protein.

In a specific embodiment, the present disclosure provides a complex of the adhesive polypeptide of the present disclosure and a functionality-imparting entity.

In one embodiment, a complex of the polypeptide of the present disclosure or a functionality-imparting entity thereof can have an ability to cause adhesion and/or immobilization of a first subject to a second subject, an ability to not self-aggregate, or both.

In one embodiment, a complex of the polypeptide of the present disclosure or a functionality-imparting entity thereof has an ability to cause monolayer adhesion of the first subject.

In a specific embodiment, the first subject and the second subject are the same. In another embodiment, the first subject and the second subject are different.

In one embodiment, at least one of the first subject or the second subject is a functionality-imparting entity. Examples of the functionality-imparting entities include, but are not limited to, such entities having functions useful in biosensors, separation and labeling of biomolecules, regenerative medicine, production of bio, organic or inorganic hybrid materials, surface modification and functionalization, fermentation industry, pharmaceutical industry, chemical product industry, manufacture of biofuel, wastewater treatment, bioremediation, and the like. In a specific embodiment, functionality-imparting entities are proteins and/or peptides. In a more specific embodiment, functionality-imparting entities are streptavidin, neutral avidin, derivatives thereof, enzymes, antibodies, fluorescent molecules, receptors, functional peptides (antibacterial, fluorescent and signaling factors, inducing factors, etc.), nucleic acid aptamers, or serum components.

In one embodiment, a complex of the polypeptide, fusion polypeptide, or functionality-imparting entity thereof of the present disclosure is used for adhesion and/or immobilization of cells. Adhered and/or immobilized cells are any cells. Examples thereof include, but are not limited to, animal cells, vegetable cells, microorganism cells, and the like.

In one embodiment, the second subject is a support. In a specific embodiment, the support is a liposome.

In one embodiment, a complex of the polypeptide, fusion polypeptide, or functionality-imparting entity thereof of the present disclosure has an ability to cause adhesion of a polypeptide of an amino acid sequence that is different from AtaA contained in the polypeptide described above or a functionality-imparting entity contained in the complex described above, to a second subject.

### (Production of adhesive polypeptides)

In one embodiment, the adhesive polypeptides and fusion polypeptides of the present disclosure are produced by expression systems using microorganisms.

The adhesive polypeptides, or fusion polypeptides thereof of the present disclosure, can be produced in *E*. *coli* as recombinant proteins in the manner commonly used in laboratories. That is, a gene encoding a fragment of AtaA or a fusion peptide obtained by fusing a peptide to the fragment is inserted at an appropriate position of an appropriate *E. coli* expression vector, such as the pET system, to transform *E. coli,* followed by adding an inducer to induce gene expression or to constitutively express under a constitutive expression promoter. The protein is often produced within the cytoplasm; however, the protein can also be secreted and produced in the periplasm. In the latter case, the gene sequence encoding the signal peptide originally possessed by the AtaA protein or the signal peptide integrated into the *E. coli* expression vector is coupled to the 5'end side encoding the protein so that the protein can be produced in the form of a signal peptide added to the amino-terminal side of the protein. Note that the signal peptide is cleaved and removed when the produced protein is secreted into the periplasm; thus, the resulting AtaA fragment or fusion protein thereof does not contain a signal peptide. The protein produced within cells is usually separated and purified from the solubilizing component, but may be obtained as an active protein by unwinding after its separation from the inclusion body. Note that the produced protein can be separated and purified by a commonly used affinity purification method using a His tag or Strep tag fused to the protein in advance. Furthermore, when a SNAP protein or the like is fused thereto, a molecule having a benzylguanyl (BG) group may be used for separation and purification. To further increase the degree of purification, ion exchange chromatography or gel filtration may be used in combination.

In the production method of adhesive polypeptides using this microorganism, detailed conditions, such as the method for expressing adhesive polypeptides, the host for expressing adhesive polypeptides and the method for purifying adhesive polypeptides secreted from the host, are to be appropriately adjusted by those skilled in the art. Furthermore, the adhesive polypeptides of the present disclosure can also be expressed using various expression systems such as, secretory production systems and cell-free expression systems, in addition to the intrabacterial expression systems of microorganisms.

The method for causing the above-mentioned microorganisms to produce the polypeptides of interest including the adhesive polypeptides of the present disclosure is merely exemplary, and those skilled in the art can prepare synthetic genes using a method commonly used in the art, while referring to the nucleic acid sequences (base sequences) or amino acid sequences of the genes of the present disclosure, thereby readily performing the design and construction of a DNA fragment to be inserted into the expression vector.

### (Use of polypeptide)

In one aspect, the adhesive polypeptide of the present disclosure is useful in a material engineering process. In another aspect, the adhesive polypeptide of the present disclosure is useful in an industry that uses a bioprocess. In a certain embodiment, the adhesive polypeptide of the present disclosure densely consolidates and immobilizes the proteins, peptides or cells, thus enabling material engineering processes such as surface modification and material design as well as bioprocesses in technical fields such as fermentation industry, pharmaceutical industry, chemical product industry, manufacture of biofuel, wastewater treatment, bioremediation, regenerative medicine, cell engineering, medical engineering, and biosensors, to be carried out efficiently.

In another aspect, the adhesive polypeptide of the present disclosure can be immobilized onto a liposome containing an enzyme or the like and utilized in a biosensor, environmental cleanup, or the like. When used especially in the environment, the adhesive polypeptides are advantageous in that they cannot grow unlike live cells so that there is hardly any risk of biohazard or ecological destruction from using a recombinant gene or protein.

### (General technology)

The molecular biological methodology, biochemical methodology, and microbiological methodology used herein are well known and commonly used in the art, which are described, for example, in Sambrook J. et al. (1989). Molecular Cloning: A Laboratory Manual, Cold Spring Harbor and its 4th Ed. (2012), Masato Okada, Kaori Miyazaki, 2011, "Protein Experiment Notes, Revised 4th Edition", First and Second Volumes, Yodosha Co., Ltd., or the like. Relevant portions thereof (which may be the entire document) are incorporated herein by reference.

### (Note)

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When required, organisms used in the following Examples were handled in compliance with the guidelines stipulated by the Nagoya University, regulatory agency, or the Cartagena Protocol. For reagents, the specific products described in the Examples were used. However, the reagents can be substituted with an equivalent product from another manufacturer (Sigma-Aldrich, Fuji Film, Wako Pure Chemical, Nacalai Tesque, R & D Systems, USCN Life Science INC, Thermo Fisher Scientific, Kanto Chemical, Funakoshi, Tokyo Chemical Industry, Merck, or the like).

### (Example 1: Search for cell surface molecules forming an aggregate with AtaA in a cell aggregation)

This Example shows cell aggregation analysis through microscope observation obtained by an experimental approach shown in the experimental model of Figure **1A****.**

### (Method)

An *Acinetobacter* sp. Tol5 strain that expresses mRFP (red fluorescent protein) and expresses a normal AtaA protein was homogenously admixed with an *Acinetobacter* sp. Tol5 strain that expresses EGFP (green fluorescent protein) and expresses a normal AtaA protein or a ΔAtaA strain. The formed cell aggregate was observed under a microscope. The resulting image was divided into small regions to calculate the ratio of cells that express EGFP.

### (Results)

Figure **1B** shows an image of a cell aggregate formed by mixing an *Acinetobacter* sp. Tol5 strain that expresses EGFP with an *Acinetobacter* sp. Tol5 strain that expresses mRFP and a histogram of the ratio of cells that express EGFP in a small section. Both fluorescent protein expressing strains express a normal AtaA protein. Figure **1C** shows an image of a cell aggregate formed by mixing an *Acinetobacter* sp. Tol5 strain that expresses mRFP and a normal AtaA protein with a ΔAtaA strain that expresses EGFP and a histogram of the ratio of cells that express EGFP in a small section. In Figure **1B****,** cells that express EGFP are homogenously present in a cell mass, and the histogram of the ratio of cells that express EGFP exhibited a peak in the middle. Meanwhile in Figure **1C****,** cells that express EGFP were not taken into the cell mass, and a histogram of the ratio of cells that express EGFP exhibited a peak at an edge.

### (Discussion)

It was demonstrated that cells that express AtaA did not form an aggregate with other cell surface molecules, but aggregated with cells that express AtaA. Specifically, it was demonstrated that cell-aggregation of cells of a Tol5 strain is due to an interaction between AtaA molecules.

### (Example 2: Search for a self-aggregation domain)

This Example searched for a domain associated with self-aggregation in an AtaA protein.

### (Method)

(1) AtaA with a structure that is deficient of Nhead (SEQ ID NO: 7), (2), (3), and (4) AtaA with a structure that is deficient of a part of Nstalk (SEQ ID NOs: 9, 11, and 13, respectively), (5) AtaA with a structure that is deficient of Chead in addition to a part of Nstalk (SEQ ID NO: 15), and (6) AtaA with a structure that is deficient of the majority of Cstalk (while the FGG domain is preserved) (SEQ ID NO: 17) were designed (Figure **2A**). Construction of each partially deficient form involves cleaving, deleting, and re-attaching at a junction between domains in a manner that does not disrupt the structure based on domain annotation, or if it is unavoidable to cleave within a domain, domains were attached so that the domain and the amino acid sequence were preserved after deletion by utilizing a repeat sequence that is present in AtaA. Furthermore, if a repeat sequence cannot be used for being within a domain, the periodicity of a coiled coil was restored after cleaving, deleting, and re-attaching by utilizing the periodicity of coiled coil.

For each partially deficient form, an expression construct was constructed by utilizing pAtaA with a cloned ataA gene, pDONR221::ataA, or a synthetic gene fragment. A 4140 strain, which is an ataA gene deficient mutant strain of Tol5, was transformed by conjugating with a donor strain *E.coli* S17-1 possessing an expression construct (Simon, R.; Priefer, U.; Puhler, A., Bio-Technol 1983, 1, (9), 784-791) to obtain a transformed strain (domain deficient strain) expressing each partially deficient AtaA. A wild-type strain expressing a full length AtaA was transformed with an mRFP gene, and a domain deficient strain was transformed with an EGFP gene to prepare strains exhibiting red and green fluorescence, respectively. These transformed strains were used to evaluate the cell aggregation property by the same approach as the method described in Example 1.

### (Results)

The results are shown in Figure **2B****.** If an *Acinetobacter* sp. Tol5 strain that expresses a normal AtaA protein is mixed with (1) an *Acinetobacter* sp. Tol5 strain that expresses an AtaA protein with a structure that is deficient of Nhead, uptake of EGFP expressing domain deficient strain cells into a cell mass was not observed. Meanwhile, domain deficient strain cells expressing a partially deficient form other than (1) were homogenously present in a cell mass. A histogram of the ratio of cells expressing EGFP exhibited a peak at the center.

### (Discussion)

(1) A Tol5 wild-type strain expressing a full length AtaA protein could not aggregate with only an *Acinetobacter* sp. Tol5 strain expressing an AtaA protein with a structure that is deficient of Nhead, but was able to aggregate with an *Acinetobacter* sp. Tol5 strain expressing an AtaA protein with a structure that is deficient of a site other than Nhead. Thus, it was demonstrated that Nhead of AtaA is essential for self-aggregation of microorganism cells, i.e., interaction between AtaAs. Specifically, it is suggested that self-aggregation among Nhead induces intermolecular interaction of AtaA.

### (Example 3: Design/Preparation of Nhead recombinant protein and analysis of folding)

In this Example, a construct for inducing an Nhead domain of AtaA to be expressed as normally folded recombinant protein was designed.

### (Method)

### (Construction of construct)

*E. coli* was induced to make Nhead, which is a functional site responsible for AtaA adhesion and self-aggregation, as a recombinant protein for the first time. Figure **3A** shows a construct design of an Nhead recombinant protein (SEQ ID NO: 19). AtaA is correctly folded, and exerts its functions, by three polypeptide chains of the same type gathering to form a homotrimer. It is extremely challenging to design a construct of a recombinant protein. It is not easy, even for a protein engineering specialist, to design a trimer to properly form and correctly fold. It was inferred that the structure of Nhead is primarily comprised of a β strand from the primary structure (amino acid sequence). It was expected that formation of a trimer through association of a β strand would be difficult. It was understood that the neck, which is present on the C-terminus side of Nhead, also needed to be included in a construct. Furthermore, a GCN4pII tag, which is a coiled-coil structure known to promote trimer formation, was attached to Nhead. This was designed to maintain the periodicity of a coiled coil by attachment to a GCN4pII tag via a coiled coil, which is a part of an FGG domain at the N-terminus side tip of Nstalk for successful attachment to GCN4pII. Furthermore, a His tag was introduced to the C-terminus side of the GCN4pII tag to facilitate separation and purification. Figure **3C** shows the amino acid sequence of the Nhead recombinant protein thus designed (corresponding to the amino acid fragment at positions 59-325 of the AtaA sequence: the amino acid fragment at positions 2-268 of SEQ ID NO: 1). The origin of the sequence is also shown therein. To prepare this construct, a gene fragment encoding amino acid 1-268 positions of SEQ ID NO: 1 was inserted into the restriction enzyme XbaI/BsaI digestion fragment of the pIBA-GCN4tri-His vector, thus preparing pIBA :: Nhead recombinant DNA (pIBA-GCN4tri-His :: ataA₅₉₋₃₂₅). The secondary structure was analyzed through a CD spectrum as an indicator to find whether a purified Nhead recombinant protein was properly folded.

### (Purification of Nhead recombinant protein)

Cultures obtained by culturing BL21 star (DE3; pIBA-GCN4tri-His :: ataA₅₉₋₃₂₅) overnight were diluted 1: 100 in a LB medium and incubated at 37°C for 3 hours. After the incubation, 0.2 µg/ml anhydrous tetracycline (AHTC) was added to the medium, followed by further incubation at 28°C for 6 hours. The cells were harvested by centrifugation at 5,000×g for 15 minutes at 4°C and resuspended in a lysis buffer (20 mM Tris-HCl, 150 mM NaCl and 20 mM imidazole, pH 9.0), followed by dissolving at 1,000 bar for 10 minutes using a high-pressure homogenizer (LAB 2000, SMT Co., Ltd.). After centrifugation at 10,000×g for 15 minutes at 4°C, the supernatant was loaded onto a Ni-NTA Superflow column (Qiagen NV) and washed twice with lysis buffer to remove unbound proteins. The bound protein was eluted with an elution buffer (20 mM Tris-HCl, 150 mM NaCl and 400 mM imidazole, pH 9.0). The eluted protein was loaded onto an anion exchange column (HiTrap Q HP, GE Healthcare) and the flow-through fraction containing the recombinant protein was dialyzed against 50 mM phosphate buffer (pH 6.0). The recombinant protein was then purified using a cation exchange column (HiTrap SP HP, GE Healthcare) in a phosphate buffer (pH 6.0) with a linear concentration gradient of 0 to 1,000 mM NaCl. The peak fraction containing the recombinant protein was concentrated by ultrafiltration using a centrifugal filter device (Amicon ultra, EMD Millipore), loaded onto a gel filtration column (HiLoad 26/60 Superdex 200 pg, GE Healthcare) equilibrated with 25 mM Tris-HCl (pH 9.0), and purified by gel filtration.

### (Analysis of folding of purified Nhead recombinant protein)

The CD spectra of purified Nhead recombinant proteins were measured using J-725 spectropolarimeter (JASCO Corporation). Parameters were set to wavelengths of 190 to 250 nm, scan rate of 100 nm/min, scan interval of 0.1 nm, response of 0.25 seconds, and bandwidth of 1.0 nm.

### (Results)

The result of analysis of CD spectra showed that a purified Nhead recombinant protein prepared by *E. coli* was not in a random coil state, but was forming a secondary structure, and was inferred as being folded (Figure **3B**). In this regard, the crystalline structure of Nhead was determined after crystallization. It was found as a result thereof that Nhead has two characteristic loops (first loop and second loop) that are not found in the head domain of other TAAs.

### (Example 4: Evaluation of a function of a loop on Nhead)

This Example investigated whether first and second loops that are characteristic to Nhead are involved in nonspecific adhesion of AtaA.

### (Method)

### (Preparation of AtaA mutant strain)

Mutants with a deletion of each loop (SEQ ID NO: 21 and 23) were prepared through inverse PCR by utilizing a cloned ataA gene fragment, and were introduced and expressed in a 4140 strain (ΔataA), which is an ataA gene deficient mutant strain of Tol5. The loop deficient mutant strains, and wild-type strain (AtaA) for comparison, ΔataA, and Nhead deficient strain (ΔNhead, SEQ ID NO: 7) were subjected to a microorganism adhesion test using a polystyrene (PS) plate and a glass plate.

### (Method)

### (Microorganism adhesion test)

Retrieved cells were washed three times with ultrapure water and suspended in a BS-N medium so that OD₆₆₀ would be 0.5. Cell suspensions (200 µL each) were placed in a well of a 96-well polystyrene (PS) or glass plate. After incubating for 2 hours at 28°C without shaking, the cell suspensions were removed with a micropipette, and each well was rinsed three times with 120 µL of BS-N medium. Immobilized cells were stained for 15 minutes with 200 µL of 0.1% crystal violet solution. After rinsing three times with 200 µL of BS-N medium, a dye was eluted from cells using 200 µL of 70% ethanol, and absorbance at 590 nm of eluate (A590) was measured with a microplate reader. The composition of the BS-N medium is described in the article (S. Ishii, J. Koki, H. Unno, K. Hori; Two Morphological Types of Cell Appendages on a Strongly Adhesive Bacterium, Acinetobacter sp. Strain Tol5, Appl. Environ. Microbiol. 70, (2004) 5026-5029) and the article (H. Watanabe, Y. Tanji, H. Unno, K. Hori; Rapid Conversion of Toluene by an Acinetobacter sp. Tol5 Mutant Showing Monolayer Adsorption to Water-Oil Interface, J. Biosci. Bioeng. 106 (2008) 226-230) .

### (Results)

Figure **4** shows results of an adhesion test. The graph in Figure **4** shows the results of measuring adhesion of microorganisms by A₅₉₀. Adhesion was significantly reduced for the first and second loop deficient strains. Accordingly, it was found that these two loops are essential for adhesion of AtaA.

### (Example 5: Evaluation of self-aggregation property of recombinant Nhead expressed by E. coli)

A sample that does not aggregate was able to be obtained upon purification of Nhead recombinant proteins. In this regard, this Example evaluated the self-aggregation property of recombinant Nhead expressed by *E. coli* through dynamic light scattering (DLS).

### (Method)

A recombinant protein was dissolved in PBS buffer so that the concentration would be 0.2 mg/ml to prepare a sample solution. 40 µL of sample solution was placed in a quartz cell, and the particle shape was measured with a DLS analyzer equipped with a He-Ne laser (wavelength of 633 nm) (Zetasizer Nano ZSP, Malvern Instruments, UK). The measurement was taken under conditions with a temperature of 25°C, equilibrium time of 120 seconds, and scattering angle of 173°.

### (Results and discussion)

Figure **5** shows the results. Recombinant Nhead expressed by *E. coli* quite unexpectedly did not exhibit a self-aggregation property. Therefore, it was revealed that recombinant Nhead expressed by *E. coli* exhibited a property that is different from Nhead on AtaA expressed in the *Acinetobacter* sp. Tol5 strain.

(Example 6: Feature of aggregate of *Acinetobacter* sp.

### Tol5 strain)

The inventor unexpectedly found that an AVL peptide has a function of interacting with first and second loops of Nhead to disintegrate an aggregate of *Acinetobacter* sp. Tol5 strain cells by AtaA. This Example observed disintegration of an aggregate of the *Acinetobacter* sp. Tol5 strain by said peptide under a microscope.

### (Method)

An aggregate of the *Acinetobacter* sp. Tol5 strain and the *Acinetobacter* sp. Tol5 strain in the presence of an AVL peptide within a flow cell were observed under a microscope. A flow cell prepared by the inventor in the manner described below was used.

### (Preparation of flow cell)

The basic structure of the flow cell system constructed in the present disclosure is a silicone tube, for delivering a cell suspension or fluid, to which a glass tube is connected for observing cells. To construct a rectangular parallelepiped flow cell system, a silicone tube with a length of 300 mm, an inner diameter of 1 mm, and an outer diameter of 2 mm was connected to either end of rectangular parallelepiped glass with a length of 50 mm and each inner diameter of 1 mm (Vitrocom), where the connections were sealed with paraffin film. The silicone tube at the entrance was connected to a syringe pump (Legato 200, KD Scientific) via a three-way stopcock (Terumo Corporation), and the fluid was sent to the cell observation part (flow cell) of the rectangular parallelepiped glass tube.

A Tol5 cell suspension suspended in a BS-N medium was allowed to flow in the flow cell described above for 30 minutes at a rate of 64 µl/min to allow an aggregate of Tol5 cells to adhere to a glass flow cell. Subsequently, a BS-N medium comprising 0.1 mM of AVL peptide was allowed to flow at the same rate. The behavior of the adhered Tol5 cell aggregate was observed under a digital microscope (Keyence VHX-200).

### (Results and discussion)

Figure **6** shows the results. Disintegration of an *Acinetobacter* sp. Tol5 strain cell aggregate by an AVL peptide was observed. An aggregate was not affected whatsoever even if a medium free of said peptide was allowed to flow at the same rate. It was found therefrom that an AVL peptide has a function of disrupting self-aggregation of Nheads of AtaA.

### (Example 7: Measurement of adhesive property of E. coli recombinant Nhead)

In this Example, adhesion of recombinant Nhead expressed by *E. coli* was measured through QCM-D.

### (Method)

A recombinant Nhead protein (SEQ ID NO: 19) was dissolved in a PBS solution at a concentration of 50 µg/ml to prepare a sample solution. As a device, Q-Sense E4 (Biolin Scientific, Sweden) was used, and a polystyrene (PS) or silica sensor was used. First, a PBS buffer was allowed to flow at a flow rate of 250 µl/minute, and the inventor waited until the device stabilized. Subsequently, the sample solution was allowed to flow for 1.5 minutes at the same flow rate. The flow was stopped, the solution was left for 15 minutes and then the sample solution was allowed to flow again for 1.5 minutes at the same flow rate. The flow was again stopped and the solution was left for 10 minutes, and then rinsed by allowing PBS buffer to flow for 1 minute at the same flow rate. Furthermore, the flow was stopped and the solution was left for 5 minutes. Subsequently, 0.1 mM AVL peptide solution was allowed to flow for 2 minutes at the same flow rate, and the solution was left for 15 minutes after stopping the flow. Finally, PBS buffer was allowed to flow for 2 minutes at the same flow rate. The flow was stopped, and the buffer was left for 10 minutes to end the measurement.

### (Results)

Figures **7A** and **7B** show the results. The Δf7/7 (Hz) value decreased to reach an equilibrium state immediately after adding Nhead no matter which of a polystyrene sensor or silica sensor was used. A change in the Δf7/7 (Hz) value was not observed even after the subsequent Nhead re-supplying step, rinsing step using a buffer, AVL peptide adding step, and second rinsing step using a buffer.

### (Discussion)

It was understood that addition of *E. coli* recombinant Nhead resulted in immediate adhesion to a sensor to reach equilibrium. Since a change in the Δf7/7 (Hz) value was not observed thereafter even after re-supplying an *E. coli* recombinant Nhead solution, it was understood that Nhead did not further adhere. In view of the above, it is conjectured that *E. coli* recombinant Nhead has an ability to adhere to, but not accumulate, on a polystyrene or silica surface. This reflects that *E. coli* recombinant Nhead does not have a self-aggregation property. It is also understood that *E. coli* recombinant Nhead adhering to the surface does not peel off from simply rinsing with a buffer. Even when an AVL peptide that disrupts self-aggregation of native Nhead was subsequently supplied, separation was not observed. It can be understood in view of this fact that *E*. *coli* recombinant Nhead does not exhibit a self-aggregation property or adhere as a laminated layer. Separation due to re-rinsing was also not observed. In view of the above, it was found that *E. coli* recombinant Nhead does not laminate, but adheres in a single layer on a solid surface. It is understood that a small variation in signals from re-supplying Nhead, rinsing, supplying an AVL peptide, and re-rinsing is noise due to the operation.

### (Example 8: Self-aggregation property and microorganism immobilization rate of cells expressing WChead)

An unpredicted and perplexing result for the researcher conducting the study was obtained up to Example 7, where Nhead, when present at the tip of AtaA on a microorganism cell, exhibits adhesive and self-aggregation properties, but a recombinant Nhead protein produced by *E*. *coli* exhibited only an adhesive property, but not a self-aggregation property. From the application aspect, such an unexpected property of a recombinant Nhead protein results in a useful function, i.e., monolayer adhesion to the material surface. In this regard, the function of domains other than Nhead was studied in more detail in order to elucidate the reason thereof. It is a known fact that Chead, when present on AtaA on a microorganism cell, is not involved in adhesive or self-aggregation properties (K. Koiwai, M. D. Hartmann, D. Linke, A. N. Lupas and K. Hori; Structural basis for toughness and flexibility in the C-terminal passenger domain of an Acinetobacter trimeric autotransporter adhesin, J. Biol. Chem. 291 (2016) 3705-3724.) However, Chead is present at a base of an AtaA fiber. It was hypothesized that at this location, it is difficult for Chead to interact with the surface or other AtaA molecule, but Chead with a similar structure (Ylhead) at a tip of an AtaA fiber in the same manner as Nhead would also exert a function of adhesion or self-aggregation. In this regard, a WChead mutant strain with Nhead of AtaA substituted with Chead was prepared. This Example measured the self-aggregation property and immobilization rate of microorganism cells expressing WChead.

### (Method)

A WChead variant was prepared to have a Chead-Nstalk-Chead-Cstalk domain group structure by deleting a full length AtaA protein Nhead domain and incorporating a Chead domain in said region (SEQ ID NO: 25). Cell adhesive properties of the WChead strain, and the wild type strain (AtaA), ΔAtaA strain, and ΔNhead strain were compared. Adhesive properties were tested by the same adhesion testing method as Figure **4****.** For the self-aggregation test, a bacterial suspension was prepared by suspending microorganism cells in a BS-N medium so that the initial OD₆₆₀ would be 0.5. Self-aggregation properties were examined through tube-settling assay by using this suspension. Detailed procedures of this method are described in M. Ishikawa, K. Shigemori, A. Suzuki, and K. Hori; Evaluation of adhesiveness of Acinetobacter sp. Tol5 to abiotic surfaces, J. Biosci. Bioeng. 113 (2012) 719-725. However, the test tube was left for 3 hours.

### (Results)

Figures **8A** and **8B** show the results. Since the self-aggregation property found in wild-type AtaA was lost in the ΔNhead deficient strain in Figure **8A****,** this again demonstrated that Nhead is associated with self-aggregation properties. Furthermore, a self-aggregation property is found when WChead is expressed. Thus, it was demonstrated that Chead, when present at the AtaA fiber tip in the same manner as Nhead, also exerts a self-aggregation property. In Figure **8B****,** microorganism adhesion found in the wild-type AtaA was reduced in the WChead expressing strain to the same extent as ΔNhead deficient strain. Thus, it was elucidated that Chead does not exhibit an adhesive property like Nhead, even when located at the tip of an AtaA fiber. Specifically, it was unexpectedly discovered that Chead does not have an adhesive property, but can exhibit an aggregation property depending on the location where it is present on an AtaA fiber, i.e., potentially has a self-aggregation function.

### (Discussion)

The passenger domain of AtaA cleaved from a fiber is known to have adhesion and self-aggregation capabilities. Such a passenger domain comprises both Nhead and Chead. It is understood that Chead on an AtaA fiber, when the AtaA fiber is cleaved from a cell, is released from steric hindrance when present on a cell surface layer, so that a latent self-aggregation function is exerted. When an AtaA fiber is present on a cell surface layer, the orientation of proteins is aligned, so that a sufficient force to induce cell aggregation can be exerted with only Nhead at the tip. However, a passenger domain fiber of AtaA cleaved from a cell loses orientation, so that the contact efficiency between Nhead decreases compared to when it is on the cell. For this reason, a self-aggregation property cannot be exerted with only Nhead. However, if Chead is present, a domain with an aggregation property would be close to both ends of a fiber, so that a self-aggregation property would be exhibited. Specifically, it is understood that an AtaA fragment cleaved from a microorganism cell exhibits a self-aggregation property when having both Nhead and Chead, but would not exhibit a self-aggregation property when only Nhead is present without Chead. Accordingly, when an AtaA fragment that does not comprise Chead or an AtaA fragment having Chead introduced with a mutation to eliminate the self-aggregation function of Chead comprises Nhead, a recombinant protein having an adhesion function, but without a self-aggregation function is produced.

### (Example 9: Immobilization of a fusion product with an Nhead fusion peptide)

An Example of immobilizing a functional protein on a material surface by utilizing a monolayer adhesion of Nhead is provided. For this purpose, a fusion peptide prepared by fusing a SNAP protein to Nhead (SEQ ID NO: 27) was designed as a model protein. Figure **9A** shows the construct design. The design involves fusing a SNAP protein between a GCN4pII tag and a His tag of the Nhead recombinant protein shown in Figure **3****.** Figure **9C** shows the sequence of this Nhead-SNAP fusion peptide. The secondary structure was analyzed through CD spectra as an indicator for finding whether a purified Nhead-SNAP fusion peptide is correctly folded.

### (Method)

A DNA construct was prepared by attaching a fragment amplified by inverse PCR from pIBA-GCN4tri-His: :ataA₅₉₋₃₂₅ to a separately prepared DNA (SNAP-DNA) fragment encoding SNAP protein by In-Fusion cloning. This was introduced into and produced by *E. coli,* and the fusion peptide was purified. The specific procedure is the same as that described in Example 3.

### (Results)

The results of CD spectrum analysis show that a purified Nhead-SNAP fusion peptide produced by *E. coli* is not in a random coil state, but forms a secondary structure (Figure **9B**).

### (Example 10: Immobilization of Nhead-SNAP fusion peptides onto various material surfaces)

This Example attempted to immobilize an Nhead-SNAP fusion peptide onto each of glass, polystyrene (PS), titanium (Ti), and polytetrafluoroethylene resin (PTFE) surfaces.

### (Method)

Figure **10A** shows the procedure of immobilization of the fusion peptide onto a surface. Holes with a 5 mm diameter were opened at a 1.5 cm interval on 4 layers of polyvinyl chloride tape (VT-196; Nichiban) applied onto a Teflon^{®} tape (Nitto Tape No. 303; Nitto Denko) with a hole punch. The Teflon tape was peeled off and applied to the target material before use to prepare a polyvinyl chloride test well with a depth of 0.8 mm on a flat material. 1 µM of Nhead-SNAP fusion peptide solution was added dropwise to the well and left for 1 hour. A sample without adding anything (Blank) and a sample added with only a SNAP protein were also prepared as negative controls. To quantify the fusion peptide immobilized on the surface, 1 µM of SNAP-Surface 488, which is a fluorescent substrate molecule of a SNAP protein that is a product fused to Nhead, was added dropwise and left for 1 hour at 37°C to covalently bind a fluorescent substrate to a SNAP protein via a benzylguanyl group. After washing 5 times with PBS, the sample was exposed to light for 120 seconds while irradiating green LED light comprising a 506 nm excitation wavelength to capture 526 nm fluorescence with a fluorescence scanner.

### (Results)

Figure **10B** shows the results. Generation of fluorescence was observed in all substrates to which an Nhead-SNAP fusion peptide was added. Thus, it was elucidated that Nhead-SNAP fusion peptides can adhere to, and be immobilized on, all materials used in the experiment via Nhead. It is also demonstrated that a SNAP protein alone cannot be immobilized onto a substrate.

### (Example 11: Immobilization of an antimicrobial peptide with an Nhead-SNAP fusion peptide)

This Example attempted to impart antimicrobial activity to a material surface by immobilizing an antimicrobial peptide with an Nhead-SNAP fusion peptide.

### (Method)

15 µl of 3 µM Nhead-SNAP fusion peptide solution was added to a 12-well highly water-repellant printed microscope slide (Matsunami Glass) and left for 1 hour at room temperature. The solution was washed three times with 20 µl of PBS, combined with 9 µM benzylguanylated (BG) CAP18 antimicrobial peptide, and incubated for 1 hour at 37°C to covalently bind the antimicrobial peptide to the SNAP protein portion of the fusion peptide. The fusion product was then washed three times with 20 µl of PBS. 15 µl of M9 medium (OD600 = 0.01) in which *Pseudomonas aeruginosa* PAO1 was suspended was added, and the mixture was left for 15 minutes at room temperature. After removing the microorganism suspension, this was washed three times with 20 µl of M9 medium. 15 µl of M9 medium was then added, and the mixture was left and was cultured for 10 hours at 37°C. After culture, the culture was washed three times with PBS. Microorganism cells were fluorescently stained for 15 minutes using SYTO9 with a final concentration of 3.34 µM. Finally, the cells were washed 5 times with PBS, and a biofilm formed on the microscope slide was observed under a confocal laser microscope.

### (Results)

Figure **11** shows the results. A BG-lated antimicrobial peptide was immobilized to suppress the formation of a biofilm of *Pseudomonas aeruginosa* only on an Nhead-SNAP-coated polystyrene surface when the peptide was added. However, formation of a biofilm of *Pseudomonas aeruginosa* could not be suppressed on a surface coated with only a SNAP protein or on an untreated surface, even after adding a BG-lated antimicrobial peptide. It was confirmed that a SNAP protein itself does not have an ability to adhere to a surface, and an antimicrobial peptide itself does not exhibit an ability to adsorb onto a surface. Accordingly, immobilization of a SNAP protein onto a surface using Nhead as an adhesion tag and attachment of a BG-lated functional molecule to SNAP enabled preparation of functional surfaces such as an antimicrobial surface.

### (Example 12: Preparation of SNAP-Nhead-GCN4pII fusion peptide having a SNAP protein on the N-terminus side)

The objective of this Example is to prepare an Nhead having a SNAP protein on the N-terminus side (SEQ ID NO: 29) and to reveal whether this would fold in a normal manner.

### (Method)

A DNA fusing (1) a base sequence upstream of an ataA gene (GA ATT CAT CTC CTA AGG AAA AGC GAT (SEQ ID NO: 5): underlined is the restriction enzyme EcoRI recognition sequence), (2) an AtaA signal peptide recognition sequence (amino acid positions 1 to 59 of SEQ ID NO: 4)-SNAP protein-glycine linker encoding base sequence, and (3) a base sequence encoding the Nhead tip (positions 7 to 35 of the base sequence of SEQ ID NO: 2) (to the restriction enzyme XmnI cleavage site) was prepared through overlap PCR by utilizing pDONR::ataA with a cloned ataA gene, and a SNAP-DNA fragment. This was attached to pDONR::ataA EcoRI or XmnI digested fragment to construct a fusion gene fragment encoding a polypeptide with a SNAP protein and a glycine linker inserted between AtaA's signal peptide recognition sequence and Nhead (or to be exact, this without the first alanine residue). This was used as a template to amplify a DNA fragment encoding Nhead and coiled coil portion (amino acid positions 3 to 268 of SEQ ID NO: 1) of SNAP protein-glycine linker-AtaA by PCR. This was inserted into a pIBA-GCN4tri-His in the same manner as Example 3 to prepare a pIBA::SNAP-Nhead fusion peptide (pIBA::SANP-AtaA₆₀₋₃₂₅). This was introduced into *E. coli* and produced as a recombinant protein. Production and purification of a SNAP-Nhead fusion peptide, and whether the purified fusion peptide is correctly folded, were checked in accordance with the Example 3.

### (Results)

Figure **12** shows the results. As a result of CD spectrum analysis, a purified SNAP-Nhead fusion peptide produced by *E. coli* is shown to be not in a random coil state, but was forming a secondary structure, and is inferred to be folded.

### (Example 13: Evaluation of the ability to immobilize and function of a SNAP-Nhead fusion peptide)

This Example evaluated the immobilization function of an antimicrobial peptide by the SNAP-Nhead fusion peptide prepared in Example 12.

### (Method)

First, BG-lated antimicrobial peptide CAP18 was reacted with a fusion peptide immobilized on a surface and covalently attached to a SNAP protein within the fusion peptide. Next, a fluorescent substrate of the SNAP protein was reacted to the immobilized fusion peptide to covalently attach the fluorescent substrate to the SNAP protein, which was open because an antimicrobial peptide could not attach thereto. The efficiency of attachment to a fusion peptide of CAP18 can be found by comparing the fluorescence intensity with fluorescence intensity from when a fluorescent substrate is reacted without reacting with CAP18 in advance. For this reason, 100 µl of fusion peptide solution (1 µM) was applied to a 96-well black polystyrene plate and left for 1 hour at room temperature to immobilize the fusion peptide onto the plate. After washing three times with 110 µl of PBS, the sample was reacted by adding 100 µl of 3 µM BG-lated CAP18 antimicrobial peptide solution and incubating for 1 hour at 37°C. After washing three times with 110 µl of PBS, 100 µl of 3 µM SNAP surface 488 was applied and reacted for 1 hour at 37°C. Finally, this was washed 5 times with 110 µl of PBS, and 485 nm exciting light was irradiated to measure the fluorescence intensity of a 535 nm wavelength with a plate reader. Meanwhile, fluorescence intensity from applying SNAP Surface 488 without the reaction step with CAP18 was also measured by the same method. This test was conducted on an Nhead-SNAP fusion peptide and the original SNAP protein as a control in addition to the SNAP-Nhead fusion peptide.

### (Results)

Figure **13** shows the results. A SNAP-Nhead fusion peptide resulted in a similar decrease in the fluorescence value as an Nhead-SNAP fusion peptide. Thus, it was concluded that a SNAP-Nhead fusion peptide has the same ability to immobilize a peptide as an Nhead-SNAP fusion peptide.

### (Discussion)

It was shown that when an Nhead recombinant protein is used as an adhesion tag, a peptide to be immobilized can be fused on either the carboxy terminus side or the amino terminus side of Nhead.

### (Example 14: Preparation of an Nhead-SNAP fusion peptide without a GCN4pII tag)

The objective of this Example is to elucidate whether a GCN4pII tag fused with the expectation of assisting in trimerization of an AtaA fragment is essential, or not required, for folding of an *E. coli* recombinant Nhead protein.

### (Method)

Figure **14A** shows the construct design (SEQ ID NO: 31) of a fusion peptide prepared in this Example. A DNA construct of interest was constructed by deleting a region encoding a GCN4pII tag through inverse PCR by utilizing a DNA construct constructed for preparing the Nhead-SNAP fusion peptide shown in Example 9. Figure **14C** shows the amino acid sequence of the construct Nhead-SNAP_ΔGCN4pII fusion peptide. The fusion peptide was purified in accordance with the method of purifying an Nhead recombinant protein described in Example 3. The CD spectra of the purified fusion peptide were measured by the same approach as Example 3.

### (Results)

The results of CD spectrum analysis show that a purified Nhead-SNAP_ΔGCN4pII fusion peptide produced by *E*. *coli* was not in a random coil state, but was forming a secondary structure, and was folded (Figure **14B**).

### (Discussion)

It is understood from the above results that a trimerized tag, i.e., GCN4pII tag, is not essential for the design and preparation of an *E. coli* recombinant Nhead protein and a fusion peptide thereof.

### (Example 15: Preparation of an Nhead-SNAP fusion peptide without a coiled coil region)

The objective of this Example is to elucidate whether an *E. coli* recombinant protein can be prepared, or a prepared construct can be folded from having activity, with only the Nhead domain region (Nhead-neck) by removing a coiled coil region from the Nhead-SNAP fusion peptide shown in Example 9.

### (Method)

Figure **15A** shows the construct design (SEQ ID NO: 33) of the fusion peptide prepared in this Example. A DNA construct of interest was constructed by deleting a region encoding a coiled coil through inverse PCR by utilizing a DNA construct constructed for preparing the Nhead-SNAP fusion peptide shown in Example 9. Figure **15C** shows the amino acid sequence of the construct Nhead-SNAP_ΔCC fusion peptide. The fusion peptide was purified in accordance with the method of purifying an Nhead recombinant protein described in Example 3. The CD spectra of the purified fusion peptide were measured by the same approach as Example 3.

### (Results)

The results of CD spectrum analysis show that a purified Nhead-SNAP_ΔCC fusion peptide produced by *E. coli* was not in a random coil state, but was forming a secondary structure, and was folded (Figure **15B**).

### (Discussion)

It is understood from the above results that a coiled coil portion is not required for the design and preparation of an *E. coli* recombinant Nhead protein and a fusion peptide thereof.

### (Example 16: Evaluation of function of various fusion peptides of Nhead and SNAP protein)

This Example evaluated the ability to immobilize for various fusion peptides of Nhead and SNAP protein prepared in Examples 14 and 15. The amount of immobilization of a fusion peptide was evaluated using a fluorescence substrate of a SNAP protein.

### (Method)

SNAP Surface 488 was attached to a fusion peptide immobilized onto a 96-well black polystyrene plate by the method in accordance with Example 13, and immobilization was quantified by measurement with a plate reader. However, unlike Example 13, the peptide was not attached to CAP18 in advance.

### (Results)

It was elucidated that an Nhead-SNAP_ΔGCN4pII fusion peptide and an Nhead-SNAP_ΔCC both have an immobilization capability that is equivalent to or greater than an Nhead-SNAP fusion peptide.

### (Discussion)

It was revealed that when a functional peptide is fused, there is no need to utilize a GCN4pII tag or need to keep a coiled coil region of AtaA. Even without them, Nhead and a fusion peptide of Nhead are correctly folded and exert a function.

### (Example 17: Application example of E. coli recombinant Nhead as a surface adhesion protein)

This Example shows that *E. coli* recombinant Nhead and streptavidin bind to form a complex.

### (Method)

In accordance with Example 5, the particle shape of *E*. *coli* recombinant Nhead alone, streptavidin (SA) alone, and a protein in a mixture of Nhead and SA was measured by DLS. For a solution of an individual protein, the concentration of sample solution was adjusted to 0.2 mg/ml in the same manner as Example 5. When two types of proteins were mixed, equal amounts of each sample solution adjusted to 0.2 mg/ml were mixed and left for 5 minutes. 40 µl of the mixture was then used for measurement.

### (Results)

Figure **17** shows the results. A peak of a single molecule is manifested at 5 to 10s of nm for both *E. coli* recombinant Nhead alone and SA alone. When these are mixed, the peak shifts to 1000s of nm. This is much larger than the simple sum of single molecules of *E. coli* recombinant Nhead and SA. This shows that large number of both protein molecules aggregated while forming a complex.

### (Example 18: Attachment of E. coli recombinant Nhead and neutral avidin)

This Example shows that *E. coli* recombinant Nhead attaches to neutral avidin to form a complex.

### (Method)

This Example was conducted using the same method as Example 17. However, SA in Example 17 was substituted with neutral avidin in this Example.

### (Results)

Figure **18** shows the results. A peak of a single molecule is manifested at 5 to 10s of nm for both *E. coli* recombinant Nhead alone and neutral avidin alone. When these are mixed, the peak shifts to 1000s of nm. This is much larger than the simple sum of single molecules of *E*. *coli* recombinant Nhead and neutral avidin. This shows that large number of both protein molecules aggregated while forming a complex.

### (Comparative Example 1: Interaction between E. coli recombinant Nhead and avidin)

This Example shows that *E. coli* recombinant Nhead does not attach to avidin.

### (Method)

This Example was conducted using the same method as Example 17. However, SA in Example 17 was substituted with avidin in this Example.

### (Results)

Figure **19** shows the results. A peak of a single molecule is manifested at 5 to 10s of nm for *E. coli* recombinant Nhead alone, avidin alone, and mixture of both proteins. This revealed that *E. coli* recombinant Nhead cannot attach to avidin and thus a complex is not formed.

### (Discussion)

It is shown that a function of forming a complex of *E*. *coli* Nhead with another protein is an interaction that specifically selects a binding partner.

### (Example 19: Immobilization of cells)

This Example shows that two types of microbial cells can be immobilized on a plate coated with *E*. *coli* recombinant Nhead on the surface.

### (Method)

Figure **20A** shows the schematics of the method. A plate was coated with *E*. *coli* recombinant Nhead. *Bacillus subtilis* or *Escherichia coli* adjusted to a certain concentration was added dropwise onto the plate and incubated for 6 hours. The plate was then washed with pure water (DW), and each cell was stained with a nuclear staining agent. Cells immobilized on the plate were observed under a confocal microscope.

### (Results)

Figure **20B** shows the results. Immobilization of cells onto the plate to which one of *Bacillus subtilis* and *Escherichia coli* was added dropwise was observed. Immobilization of cells was not observed on a plate that was not coated with Nhead or a plate to which BSA was added dropwise instead of Nhead.

### (Example 20: Preparation and investigation of liposome prepared by immobilizing an Nhead-SNAP fusion peptide on the surface layer (Nhead surface layer modififed liposome))

### (Method)

### (Preparation of an Nhead surface layer modified liposome)

This liposome was prepared by reacting the Nhead-SNAP fusion peptide shown in Example 9 with a benzylguanyl (BG)-lated liposome. The BG-lated liposome was prepared as follows.

The inside of a round-bottom flask containing 300 µl of 50 mg/ml egg yolk phosphatidylcholine (COATSOME NC-50 (EPC), Yuka Sangyo) dissolved in chloroform was rotated while evacuating for 1 hour. A lipid film was hydrated with buffer A (10 mM HEPES, pH 7.6, 50 mM, potassium glutamate) or buffer B (10 mM Tris-HCl (pH 9.0)) supplemented with 25 µM of Alexa Fluor 647 to obtain 300 µL of 50 mg/ml lipid solution. Samples with GUS were used by adding 2 µM of GUS to buffer A. 1 mg/mL BSA was added to buffer B for samples subjected to observation under an electron microscope. These lipid solutions were sonicated for 10 minutes and vortexed for 10 seconds. The lipid solutions were further subjected to 5 freeze-thaw cycles. Next, a liposome suspension was extruded out with a mini extruder (Avanti Polar Lipids, Alabaster, AL, USA) by using a 0.8 µm VCTP Isopore membrane filter at room temperature. 1200 µL of buffer A or buffer B was added to 300 µL of giant unilamellar vesicle (LUV) prepared using buffer A or B, respectively. The solution was centrifuged for 30 minutes at 20,000 × g and washed by replacing the supernatant with 1200 µL of buffer A or B. The washing step was repeated 4 times.

A BG liposome was prepared as follows. Specifically, 14 µl of 2mM BG-DSPE (PEG200) dissolved in chloroform was rotated for 15 minutes while evacuating in a glass micro-test tube and hydrated with buffer A or B. The BG-DSPE solution was then added to the LUV solution so that the final concentration would be 93 µM, incubated for 20 hours at room temperature, and washed 4 times as described above.

A liposome (LUV) solution and Nhead-SNAP fusion peptide solution (10 µM) were mixed within a 1.5 mL low adsorption tube (MS-4215M; Sumitomo Bakelite). A BG-lated LUV liposome was reacted with the fusion peptide by incubation for 30 minutes at 37°C to obtain an Nhead modified liposome. The liposome was centrifuged at 8000 × g at 4°C for 5 minutes. The supernatant was removed. Pellets of the liposome were resuspended in 50 µl of 10 mM Tris-HCl (pH 9.0) to obtain an Nhead surface layer modified liposome solution.

### (Method)

### (Investigation of an Nhead surface layer modified liposome)

The particle shapes of modified liposome and Nhead surface layer modified liposome were measured by DLS to investigate whether the modified liposomes were properly prepared. The DLS measurement method was the same method shown in Example 5.

### (Results and discussion)

Figure **21** shows the results. The diameter peak was found at a particle size of about 90 nm for *E. coli* recombinant Nhead surface layer modified liposomes, whereas the diameter peak was found at a particle size of about 60 nm for normal liposomes without surface layer modification. It was found from this result that immobilization of E. *coli* recombinant Nhead-SNAP onto a liposome surface layer results in an increase in the liposome diameter due to surface layer modification, but not in aggregation of liposomes to result in monodispersion. This is because *E*. *coli* recombinant Nhead and a SNAP protein fusion peptide thereof do not have an aggregation property.

### (Example 21: Analysis of an adhesive property of an Nhead surface layer modified liposome)

### (Method)

45 µl of liposome suspension (concentration of Nhead-SNAP fusion peptide: equivalent to 1 µM or 10 µM) was added dropwise to a 96-well polystyrene plate (Becton, Dickinson and Company) and mixed with 5 µl of 10× phosphate buffered saline (1.37 M NaCl, 27 mM KCl, 81 mM Na₂HPO₄·7H₂O, 14.7 mM KH₂PO₄). The plate containing the liposome suspension was centrifuged at room temperature for 30 minutes at 700 g to allow adhesion of liposome particles onto the surface of the plate. To remove unattached liposomes, a well was washed 2 to 4 times with a buffer (9.0 mM Tris-HCl (pH 9.0), 137 mM NaCl, 2.7 mM KCl, 8.1 mM Na₂HPO₄·7H₂O, 1.47 mM KH₂PO₄), and then 50 µl of 10 mM Tris-HCl buffer (pH 9.0) was added to the well. Adhesion of liposome was evaluated by measuring fluorescent signals of AF647 encapsulated within the liposome by using a microplate reader (ARVO X3; PerkinElmer). Fluorescent signals were quantified in top reading mode to improve the signal-to-noise ratio. When detecting fluorescence from adhering liposomes, the bottom surface was masked with a black plastic tape.

### (Results and discussion)

Figure **22** shows the results. Nhead concentration dependent adhesion of BG-lated liposomes mixed with Nhead-SNAP to a polystyrene plate was observed. However, a liposome that is not BG-lated prepared as a comparative example was not modified with Nhead on the surface layer, so that adhesion thereof to the plate was not observed.

### (Example 22: Preparation and investigation of a liposome prepared by immobilizing an Nhead Nstalk (NhNs)-SNAP fusion peptide onto the surface layer (Nhead Nstalk fiber surface layer modified liposome))

This Example prepared and investigated a liposome prepared by immobilizing an Nhead Nstalk (NhNs)-SNAP fusion peptide onto the surface layer.

### (Method)

### (Preparation of an NhNs-SNAP fusion peptide)

A DNA construct of pIBA-GCN4tri-strept-tag::NhNs-SNAP was prepared by utilizing the Nhead-SNAP fusion peptide shown in Example 9 and pDONR::ataA. An *E. coli* BL21 (DE3) strain was transformed therewith. The resulting recombinant *E. coli* was cultured in an LB medium comprising 100 µg/ml ampicillin at 37°C until OD₆₀₀ would be 0.5 to 0.7. After adding 0.2 µg/ml anhydrotetracycline, the recombinant *E*. *coli* was further cultured for 16 hours at 18°C to produce an NhNs-SNAP fusion peptide (SEQ ID NO: 35). Figure **23** shows a schematic diagram of the fusion peptide. The top row shows the full length of an AtaA protein, and the bottom row shows an NhNs-SNAP fusion peptide. An NhNs fiber surface layer modified liposome was prepared as described below by using BG-lated LUV and a cell lysate of recombinant *E. coli* that produced the fusion peptide therein.

### (Preparation of a liposome prepared by immobilizing an NhNs-SNAP fusion peptide on the surface layer)

A BG-lated liposome suspension was mixed with 2.0 mg/mL of cell extract of *E. coli* BL21 (DE3) transformant that has produced an NhNs-SNAP fusion peptide. By using a rotor, the mixture was incubated for 1 hour at 4°C and then centrifuged for 10 minutes at 15,000 g to allow NhNs fiber surface layer modified liposome particles to precipitate. The precipitated liposome particles were washed twice with 10 mM Tris-HCl (pH 9.0) buffer and resuspended in the same buffer.

### (Observation of the prepared NhNs fiber surface layer modified liposome)

The resulting NhNs fiber surface layer modified liposome was observed under an electron microscope to examine the modification of the liposome. A suspension of the liposome was diluted 50-fold in 10 mM Tris-HCl buffer (pH 9.0). The liposome was allowed to adsorb to a carbon coated copper film (400 mesh) and stained for 10 seconds with a 2% phosphotungstic acid solution (pH 7.0). A grid was then vacuum dried for 10 minutes. The grid was observed at 100 kV of acceleration voltage by using a transmission electron microscope (JEM-1400 plus, JEOL). A digital image (3296 × 2472 pixels) was captured with a CCD camera (EM-14830RUBY2, JEOL).

### (Results)

Figure **24** shows the results. The state in which an Nhead Nstalk fiber is immobilized on the surface of a liposome, and the surface layer of the liposome is modified with the fusion peptide was observed under an electron microscope.

### (Example 23: Evaluation of dispersion/aggregation of Nhead Nstalk fiber modified liposomes)

### (Method)

Dispersion/aggregation was measured by the same procedure as Examples 5 and 17 by DLS. However, measurement was also taken for an *Acinetobacter* sp. Tol5 strain and ΔataA mutant strain for comparison. A bacterial cell culture was retrieved by centrifugation at 8,000 g, washed 5 times with pure water, adjusted with pure water to OD₆₆₀ = 0.05, and measured.

### (Results and discussion)

Figure **25** shows the results of Example 23. It was elucidated in view of the results of Figure **25A** that the distribution of sizes of bacterial cells differs in the presence of AtaA from the absence thereof. The majority of sizes for the *Acinetobacter* sp. Tol5 strain was about 1720 nm, which was greater by about 440 nm compared to about 1280 nm for ΔataA. This corresponds to the size of native AtaA (225 nm × 2). As shown in Figure **25B****,** it can be understood that aggregates precipitated in 100 mM KCl solution, so that the solution was transparent, while cells were suspended without self-aggregation in pure water, so that the solution was opaque. Specifically, Tol5 cells with native AtaA do not self-aggregate in pure water. Thus, the small peak found near 5500 nm seen in Tol5 of Figure **25A** does not indicate an aggregate of Tol5 strain. It is inferred in view of the diameter that this is a small cell population of about 10 cells that are weakly entangled.

In view of the results in Figure **25C****,** the diameter of an NhNs fiber surface layer modified liposome was about 1280 nm, which is greater than the diameter of about 825 nm of an unmodified liposome by 455 nm. This is about 2-fold of the length 180 nm of Nhead Nstalk. It was also elucidated that an NhNs fiber surface layer modified liposome does not exhibit a self-aggregation property. It is understood that the peak similarly seen as Tol5 of Figure **25A** near 5000 nm is also not an aggregate of NhNs fiber surface layer modified liposomes.

### (Example 24: Adhesion assay for Nhead Nstalk fiber modified liposomes and enzymatic reaction using an adhesive liposome)

This Example shows adhesion of an NhNs fiber surface layer modified liposome and an enzymatic reaction using an immobilized liposome.

### (Method)

### (Adhesion assay)

The assay was conducted by the same method as Example 20. However, adhesion to a 96-well glass bottom plate (Matsunami Glass) in addition to a polystyrene plate was evaluated.

### (Enzymatic reaction)

2 µM β glucuronidase (GUS) was encapsulated upon liposome preparation for an enzymatic reaction. A liposome was immobilized on a plate to measure enzymatic activity by the following method.

For an enzymatic assay using GUS encapsulated into a liposome adhering on the plate surface, a liposome suspension was diluted 50-fold with 10 mM Tris-HCl buffer (pH 9.0), and 50 µL of the suspension was placed on a polystyrene well plate. Liposomes were allowed to adhere on the plate surface by centrifugation. After washing and removing unimmobililzed liposomes, 50 µL of 10 mM Tris-HCl buffer comprising 10 µM TokyoGreen-β GlcU (Goryo Chemical) as a substrate of GUS was added to each well. A hydrolytic reaction was detected as a fluorescent signal at the indicated timing by using a microplate reader. The excitation wavelength and fluorescence wavelength were 485 nm and 535 nm, respectively.

### (Results)

Figure **26** shows the results of Example 24. Significant fluorescence was measured when an NhNs fiber surface layer modified liposome was obtained by mixing a BG-lated liposome with a cell lysate in both Figure **26A** (polystyrene plate) and Figure **26B** (glass plate). The fluorescence increased with an increase in the protein concentration of the cell lysate. In contrast, fluorescence was not observed for a control unmodified liposome without a BG group modification (Egg PC liposome).

Figure **26C** shows the results of measuring enzymatic activity. Significant fluorescence indicating an enzymatic reaction was observed in a well where an NhNs fiber surface layer modified liposome was immobilized.

### (Discussion)

It is demonstrated that modification of a liposome surface layer with Nhead Nstalk enables immobilization of a liposome on various material surfaces, and a chemical reaction can be performed with an enzyme encapsulated in the immobilized liposome.

### (Example 25: Analysis of self-aggregation and adhesive properties using a modified AtaA polypeptide)

This Example compares self-aggregation and adhesive properties by using various AtaA polypeptides.

### (Method)

Expression constructs encoding various domain deficient forms, variants, and/or mutants with a length of 237 amino acids or more and 3364 amino acids or less derived from an AtaA protein were designed by using the same method as the Examples described above, or experimental approach conventionally performed in the art that is known to those skilled in the art or a commercially available kit. Self-aggregation and/or adhesive properties of these various deficient forms, variants, or mutants were analyzed by the same method as the Examples described above.

### (Example 26: Preparation of a fusion peptide with a tag and/or protein other than a SNAP protein)

In this Example, a SpyTag/SpyCatcher system was introduced to an immobilization technology to establish a technology for immobilizing a protein onto a material surface by preparing a fusion protein with a tag and/or protein other than a SNAP protein.

### (Method)

### (Construction of SpyCatcher-Nhead expressing plasmid)

A DNA fragment comprising a region other than a SNAP tag was amplified by PCR using pIBA-SNAP-AtaA₅₉₋₃₂₅-GCN4tri-His as a template, and a DNA fragment comprising SpyCatcher was amplified by PCR using pDEST14-SpyCatcher002 (Addgene) as a template. These fragments were attached by NEBuilder HiFi DNA Assembly to construct pIBA-SpyCatcher-AtaA₅₉₋₃₂₅-GCN4tri-His (SEQ ID NO: 36) (Figure **27**)

### Primers used

1. GGATCCAGTGGTAGCGGAGGAGGTGGAGGTGCTAC (SEQ ID NO: 38)
2. GATGTCGTAATCCATTTTTTGCCCTCGTTATCTAG (SEQ ID NO: 39)
3. GATTACGACATCCCAACG (SEQ ID NO: 40)
4. GCTACCACTGGATCCAGTATG (SEQ ID NO: 41)

### (Construction of SpyTag-GFP expressing plasmid)

DNA fragments comprising GFP and a vector region were each amplified by PCR using pIVEX-SNAP-GFP (Addgene) as a template. The fragments were attached by NEBuilder HiFi DNA Assembly to construct pIVEX-SpyTag-GFP-his (SEQ ID NO: 37) (Figure **28**).

### Primers used

1. GGTCATCACCACCATCATCATTAATGAGGATCCGGCTG (SEQ ID NO: 42)
2. AGGCGTCCACCATCACGATAGTAGGCACCATGGAGCCCAGGCCCA (SEQ ID NO: 43)
3. TGATGGTGGACGCCTACAAGCGTTACAAGGGCTTGGGAAGCGGCCG (SEQ ID NO: 44)
4. ATGGTGGTGATGACCGCCTCCTTTGTAGAGCTCATCCATG (SEQ ID NO: 45)

### (Expression and purification of SpyCatcher-Nhead (SEQ ID NO: 36))

20 ml of bacterial culture cultured overnight at 37°C was seeded in a 2L LB medium (100 µg/ml Amp). After shake culture for 3 hours at 37°C, AHTC was added as an inducer so that the concentration would be 200 µg/l, and the bacteria were subjected to shake culture for 4 hours at 28°C. Bacteria were harvested by centrifugation (6,000 rpm, 10 min, 4°C, R12A6 rotor; Hitachi Koki). The bacteria were suspended in 200 ml of lysis buffer (25 mM Tris-HCl, 150 mM NaCl, 20 mM Imidazole, pH 9.0). The suspended bacteria were crushed for 10 minutes at 1000 bar with a French press (LAB2000; SMT COMPANY) and centrifuged (9,000 rpm, 10 min, 4°C, R12A6 rotor). The supernatant of the crushed solution was passed through Ni-NTA Superflow beads (Qiagen) equilibriated with a lysis buffer, and then 10 ml of wash buffer (25 mM Tris-HCl, 150 mM NaCl, 50 mM Imidazole, pH 9.0) and 50 ml of elution buffer (25 mM Tris-HCl, 150 mM NaCl, 400 mM Imidazole, pH 9.0) were passed through to elute out the proteins adsorbed to a column. The retrieved protein solution was subjected to anion exchange column chromatography using a HiTrap Q HP column (column volume 5 ml; GE Healthcare). As the buffer, 25 mM Tris-HCl (pH 9.0) was used for elution over a concentration gradient of 0-2M NaCl. An elution fraction comprising SpyCatcher-Nhead was collected and dialyzed to 50 mM Phosphate-NaOH (pH 6.0) at 4°C by using a dialysis tube (Standard RC Tubing MWCO: 12-14 kD; Spectra/por 4 Dialysis Membrane). The solution was collected from the tube and subjected to cation exchange column chromatography using a HiTrap SP HP column (column volume 5 ml; GE Healthcare). As the buffer, 50 mM Phosphate-NaOH (pH 6.0) was used for elution over a concentration gradient of 0-2M NaCl. An elution fraction comprising SpyCatcher-Nhead was collected and subjected to gel filtration column chromatography in a Superdex 200 column (column volume 320 ml; GE Healthcare) by using 25 mM Tris-HCl (pH 9.0). An elution fraction comprising SpyCatcher-Nhead was collected and concentrated through ultrafiltration using Amicon Ultra-15 Centrifugal filters Ultracel-10k (Merck MILLIPORE), and the buffer was exchanged with 25mM Tris-HCl (pH 7.0). The concentration of proteins after concentration was measured through a BCA assay (Pierce BCA Protein Assay Kit; Thermo Fisher Scientific).

### (Expression and purification of SpyTag-GFP (SEQ ID NO: 37))

20 ml of bacterial culture cultured overnight at 37°C was seeded in a 2L LB medium (100 µg/ml Amp). After shake culture for 7 hours at 37°C, isopropyl-β-thiogalactopyranoside (IPTG) was added as an inducer to be 1 mM, and the bacteria were subjected to shake culture for 3 hours at 37°C. Bacteria were harvested by centrifugation (6,000 rpm, 10 min, 4°C, R12A6 rotor; Hitachi Koki). The bacteria were suspended in 200 ml of lysis buffer (25 mM Tris-HCl, 150 mM NaCl, 20 mM Imidazole, pH 9.0). The suspended bacteria were crushed for 10 minutes at 1000 bar with a French press (LAB2000; SMT COMPANY) and centrifuged (9,000 rpm, 10 min, 4°C, R12A6 rotor). The supernatant of crushed solution was passed through Ni-NTA Superflow beads (Qiagen) equilibriated with a lysis buffer, and then 10 ml of wash buffer (25 mM Tris-HCl, 150 mM NaCl, 50 mM Imidazole, pH 9.0) and 50 ml of elution buffer (25 mM Tris-HCl, 150 mM NaCl, 400 mM Imidazole, pH 9.0) were passed through to elute out the proteins adsorbed to a column. The retrieved protein solution was subjected to anion exchange column chromatography using HiTrap Q HP column (column volume 5 ml; GE Healthcare). As the buffer, 25 mM Tris-HCl (pH 9.0) was used for elution over a concentration gradient of 0-2M NaCl. A pass-through fraction comprising SpyCatcher-Nhead was collected and subjected to gel filtration column chromatography in a Superdex 200 column (column volume 320 ml; GE Healthcare) by using 25 mM Tris-HCl (pH 9.0). An elution fraction comprising SpyCatcher-Nhead was collected and concentrated through ultrafiltration using Amicon Ultra-15 Centrifugal filters Ultracel-10k (Merck MILLIPORE), and the buffer was exchanged with 25 mM Tris-HCl (pH 7.0). The concentration of proteins after concentration was measured through a BCA assay (Pierce BCA Protein Assay Kit; Thermo Fisher Scientific).

### (SpyTag-GFP and SpyCatcher-Nhead attachment test)

Nhead-SpyCatcher and SpyTag-GFP-his were each adjusted to 1 µM by using 25 mM Tris-HCl (pH7). 20 µl of Nhead-SpyCatcher solution and 20 µl of SpyTag-GFP-his solution were mixed and left for 5 minutes at 37°C. The change in mobility was then studied through SDS-PAGE or CBB staining (Figure **29**).

### (Immobilization of SpyTag-GFP to a polystyrene plate using SpyCatcher-Nhead)

Nhead-SpyCatcher, SpyTag-GFP-his, and Nhead were each adjusted to 0.2 µM by using PBS. 100 µl of Nhead-SpyCatcher solution, Nhead solution, or PBS was added dropwise to a 96-well black polystyrene plate for fluorescence measurement (655086; Greiner Bio-One) and left for 1 hour at 28°C. The solution was removed from each well by pipetting and then washed twice with 100 µl of PBS. 100 µl of SpyTag-GFP-his solution was added dropwise into the well after washing, left for 5 minutes at 37°C, and then each well was washed 5 times with 100 µl of PBS. 100 µl of PBS was added to the well after washing, and fluorescence intensity of excitation wavelength 485 nm/fluorescence wavelength 535 nm was measured with a plate reader (ARVO X3; Perkin Elmer) (Figure **30**).

### (Results)

Figure **30** shows the results. It was demonstrated that a protein can be immobilized onto a material surface via Nhead even in a system using a SpyTag/SpyCatcher system.

### (Note)

As described above, the present disclosure is exemplified by the use of its preferred embodiments. However, the above descriptions and examples are provided for purposes of illustration only and not for the purpose of limiting the present disclosure. Accordingly, it is understood that the present invention is not limited to the embodiments or examples specifically described herein and the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-132488 filed on July 18, 2019 with the Japan Patent Office. It is understood that the entire content thereof is incorporated herein by reference.

### [Industrial Applicability]

The present disclosure has utility in industries that use bioprocesses.

### [Sequence Listing Free Text]

SEQ ID NO: 1: amino acid sequence of AtaA fragment including methionine at the starting position, excluding the signal peptide
SEQ ID NO: 2: nucleic acid sequence of AtaA fragment including methionine at the starting position, excluding the signal peptide
SEQ ID NO: 3: full-length nucleic acid sequence of AtaA SEQ ID NO: 4: full-length amino acid sequence of AtaA
SEQ ID NO: 5: sequence of a primer used in cloning ataA
SEQ ID NO: 6: nucleic acid sequence of AtaA with a structure that is deficient of Nhead (Example 2)
SEQ ID NO: 7: amino acid sequence of AtaA with a structure that is deficient of Nhead (Example 2)
SEQ ID NO: 8: nucleic acid sequence of AtaA with a structure that is deficient of a part of Nstalk (Example 2)
SEQ ID NO: 9: amino acid sequence of AtaA with a structure that is deficient of a part of Nstalk (Example 2)
SEQ ID NO: 10: nucleic acid sequence of AtaA with a structure that is deficient of a part of Nstalk (Example 2)
SEQ ID NO: 11: amino acid sequence of AtaA with a structure that is deficient of a part of Nstalk (Example 2)
SEQ ID NO: 12: nucleic acid sequence of AtaA with a structure that is deficient of a part of Nstalk (Example 2)
SEQ ID NO: 13: amino acid sequence of AtaA with a structure that is deficient of a part of Nstalk (Example 2)
SEQ ID NO: 14: nucleic acid sequence of AtaA with a structure that is deficient of Chead in addition to a part of Nstalk (Example 2)
SEQ ID NO: 15: amino acid sequence of AtaA with a structure that is deficient of Chead in addition to a part of Nstalk

### (Example 2)

SEQ ID NO: 16: nucleic acid sequence of AtaA with a structure that is deficient of the majority of Cstalk (while the FGG domain is preserved) (Example 2)
SEQ ID NO: 17: amino acid sequence of AtaA with a structure that is deficient of the majority of Cstalk (while the FGG domain is preserved) (Example 2)
SEQ ID NO: 18: nucleic acid sequence of AtaA₅₉₋₃₂₅-GCN4pII-His (Example 3)
SEQ ID NO: 19: amino acid sequence of AtaA₅₉₋₃₂₅-GCN4pII-His

### (Example 3)

SEQ ID NO: 20: nucleic acid sequence of a Δ1st loop variant of AtaA (Example 4)
SEQ ID NO: 21: amino acid sequence of a Δ1st loop variant of AtaA (Example 4)
SEQ ID NO: 22: nucleic acid sequence of a Δ2nd loop variant of AtaA (Example 4)
SEQ ID NO: 23: amino acid sequence of a Δ2nd loop variant of AtaA (Example 4)
SEQ ID NO: 24: nucleic acid sequence of a WChead variant of AtaA (Example 8)
SEQ ID NO: 25: amino acid sequence of a WChead variant of AtaA (Example 8)
SEQ ID NO: 26: nucleic acid sequence of AtaA₅₉₋₃₂₅-GCN4pII-SNAP-His (Example 9)
SEQ ID NO: 27: amino acid sequence of AtaA₅₉₋₃₂₅-GCN4pII-SNAP-His (Example 9)
SEQ ID NO: 28: nucleic acid sequence of SNAP-AtaA₆₀₋₃₂₅-GCN4pII-His (Example 12)
SEQ ID NO: 29: amino acid sequence of SNAP-AtaA₆₀₋₃₂₅-GCN4pII-His (Example 12)
SEQ ID NO: 30: nucleic acid sequence of AtaA₅₉₋₃₂₅-SNAP-His

### (Example 14)

SEQ ID NO: 31: amino acid sequence of AtaA₅₉₋₃₂₅-SNAP-His

### (Example 14)

SEQ ID NO: 32: nucleic acid sequence of AtaA₅₉₋₃₁₄-GCN4pII-SNAP-His (Example 15)
SEQ ID NO: 33: amino acid sequence of AtaA₅₉₋₃₁₄-GCN4pII-SNAP-His (Example 15)
SEQ ID NO: 34: nucleic acid sequence of NhNs-SNAP (Example 22)
SEQ ID NO: 35: amino acid sequence of NhNs-SNAP (Example 22)
SEQ ID NO: 36: amino acid sequence of SpyCatcher-Nhead

### (Example 26)

SEQ ID NO: 37: amino acid sequence of SpyTag-GFP (Example 26)
SEQ ID NO: 38: sequence of a primer used for constructing a SpyCatcher-Nhead expressing plasmid (Example 26)
SEQ ID NO: 39: sequence of a primer used for constructing a SpyCatcher-Nhead expressing plasmid (Example 26)
SEQ ID NO: 40: sequence of a primer used for constructing a SpyCatcher-Nhead expressing plasmid (Example 26)
SEQ ID NO: 41: sequence of a primer used for constructing a SpyCatcher-Nhead expressing plasmid (Example 26)
SEQ ID NO: 42: sequence of a primer used for constructing a SpyTag-GFP expressing plasmid (Example 26)
SEQ ID NO: 43: sequence of a primer used for constructing a SpyTag-GFP expressing plasmid (Example 26)
SEQ ID NO: 44: sequence of a primer used for constructing a SpyTag-GFP expressing plasmid (Example 26)
SEQ ID NO: 45: sequence of a primer used for constructing a SpyTag-GFP expressing plasmid (Example 26)

## Claims

1. A polypeptide comprising a fragment of the amino acid sequence set forth in SEQ ID NO: 1 or a variant sequence thereof, wherein the polypeptide does not have a self-aggregation property and has an adhesive property.

2. The polypeptide according to claim 1, comprising a sequence corresponding to amino acid positions 22 to 50 and 161 to 175 of the amino acid sequence set forth in SEQ ID NO: 1 or a variant sequence thereof, and one or more amino acid substitutions, additions, deletions, or combinations thereof in a range of a sequence corresponding to amino acid positions 2847 to 3093 of the amino acid sequence set forth in SEQ ID NO: 1.

3. The polypeptide according to claim 1 or 2, wherein the range of a sequence corresponding to amino acid positions 2847 to 3093 of the amino acid sequence set forth in SEQ ID NO: 1 is a range corresponding to amino acid positions 2855 to 3093 of the amino acid sequence set forth in SEQ ID NO: 1.

4. The polypeptide according to any one of claims 1 to 3 wherein the entire sequence corresponding to amino acid positions 2855 to 3093 of the amino acid sequence set forth in SEQ ID NO: 1 is deleted.

5. The polypeptide according to claim 1, which is
(1) a polypeptide comprising an amino acid sequence corresponding to amino acid positions 1 to 2854 of the amino acid sequence set forth in SEQ ID NO: 1 or a variant sequence thereof or a fragment thereof,
(2) a polypeptide comprising an amino acid sequence corresponding to amino acid positions 1 to 2846 of the amino acid sequence set forth in SEQ ID NO: 1 or a variant sequence thereof or a fragment thereof,
(3) a polypeptide comprising an amino acid sequence corresponding to amino acid positions 1 to 268 of the amino acid sequence set forth in SEQ ID NO: 1 or a variant sequence thereof or a fragment thereof, or
(4) a polypeptide comprising an amino acid sequence corresponding to amino acid positions 1 to 257 of the amino acid sequence set forth in SEQ ID NO: 1 or a variant sequence thereof or a fragment thereof.

6. The polypeptide according to claim 5, consisting of an amino acid sequence corresponding to amino acid positions 1 to 2854 of the amino acid sequence set forth in SEQ ID NO: 1.

7. The polypeptide according to claim 5, consisting of an amino acid sequence corresponding to amino acid positions 1 to 2846 of the amino acid sequence set forth in SEQ ID NO: 1.

8. The polypeptide according to claim 5, consisting of an amino acid sequence corresponding to amino acid positions 1 to 268 of the amino acid sequence set forth in SEQ ID NO: 1.

9. The polypeptide according to claim 5, consisting of an amino acid sequence corresponding to amino acid positions 1 to 257 of the amino acid sequence set forth in SEQ ID NO: 1.

10. The polypeptide according to any one of claims 1 to 9, wherein the variant sequence comprises an alteration by a conservative substitution.

11. The polypeptide according to any one of claims 1 to 10, which is derived from an Acinetobacter bacterium.

12. The polypeptide according to claim 11, wherein the Acinetobacter bacterium is of an Acinetobacter sp. Tol5 strain.

13. The polypeptide according to any one of claims 1 to 12, further comprising an amino acid sequence that is different from SEQ ID NO: 1.

14. The polypeptide according to claim 13, wherein the amino acid sequence that is different is fused with the fragment.

15. A complex of the polypeptide according to any one of claims 1 to 14 and a functionality-imparting entity.

16. A composition for use in causing adhesion and/or immobilization of a first subject to a second subject without self-aggregation, comprising the polypeptide according to any one of claims 1 to 14 or the complex according to claim 15.

17. The composition according to claim 16 for use in causing monolayer adhesion of the first subject to the second subject.

18. The composition according to claim 16 or 17, wherein the first subject and the second subject are the same.

19. The composition according to claim 16 or 17, wherein the first subject and the second subject are different.

20. The composition according to any one of claims 16 to 19, wherein at least one of the first subject or the second subject is a functionality-imparting entity.

21. The composition according to claim 20, wherein the functionality-imparting entity is a peptide and/or a protein.

22. The composition according to claim 21, wherein the functionality-imparting entity is streptavidin, neutral avidin, or a variant thereof.

23. The composition according to claim 20, wherein the functionality-imparting entity is a cell.

24. The composition according to any one of claims 16 to 23, wherein the second subject is a support.

25. The composition according to claim 24, wherein the support is a liposome.

26. A composition for use in causing adhesion and/or immobilization of a first subject to a second subject without self-aggregation, comprising the polypeptide according to claim 13 or 14 or the complex according to claim 15, wherein the first subject is the peptide comprising the different amino acid sequence or the functionality-imparting entity.

27. A method for use in causing adhesion and/or immobilization of a first subject to a second subject, comprising causing the polypeptide according to any one of claims 1 to 14 or the complex according to claim 15 to act on the first and/or second subject.

28. The method according to claim 27 for use in causing monolayer adhesion of the first subject to the second subject.

29. The method according to claim 27 or 28, wherein the first subject and the second subject are the same.

30. The method according to claim 27 or 28, wherein the first subject and the second subject are different.

31. The method according to any one of claims 27 to 30, wherein at least one of the first subject or the second subject is a functionality-imparting entity.

32. The method according to claim 31, wherein the functionality-imparting entity is a peptide and/or a protein.

33. The method according to claim 32, wherein the functionality-imparting entity is streptavidin, neutral avidin, or a variant thereof.

34. The method according to claim 31, wherein the functionality-imparting entity is a cell.

35. The method according to any one of claims 27 to 34, wherein the second subject is a support.

36. The method according to claim 35, wherein the support is a liposome.

37. A method for use in causing adhesion and/or immobilization of the peptide comprising the different amino acid sequence or the functionality-imparting entity to a second subject without self-aggregation, comprising causing the polypeptide according to claim 13 or 14 or the complex according to claim 15 to act on the second subject.

38. A support on which the polypeptide according to any one of claim 1 to 14 or the complex according to claim 15 is immobilized.

39. The support according to claim 38, wherein the support is selected from the group consisting of a lipid bilayer structure, glass, silica, silicon, ceramic, silicon dioxide, plastic, metal, titanium, acrylic material, and naturally-occurring and synthetic polymers.

40. Use of the polypeptide according to any one of claims 1 to 14 or the complex according to claim 15 for causing adhesion and/or immobilization of a first subject to a second subject without self-aggregation.

41. The use according to claim 40, wherein the use is use for causing monolayer adhesion of the first subject to the second subject.

42. The use according to claim 40 or 41, wherein the first subject and the second subject are the same.

43. The use according to claim 40 or 41, wherein the first subject and the second subject are different.

44. The use according to any one of claims 40 to 43, wherein at least one of the first subject or the second subject is a functionality-imparting entity.

45. The use according to claim 44, wherein the functionality-imparting entity is a peptide and/or a protein.

46. The use according to claim 45, wherein the functionality-imparting entity is streptavidin, neutral avidin, or a variant thereof.

47. The use according to claim 44, wherein the functionality-imparting entity is a cell.

48. The use according to any one of claims 40 to 47, wherein the second subject is a support.

49. The use according to claim 48, wherein the support is a liposome.

50. Use of the polypeptide according to claims 13 or 14 or the complex according to claim 15 for causing adhesion and/or immobilization of a first subject to a second subject without self-aggregation, wherein the first subject is the peptide comprising the different amino acid or the functionality-imparting entity.
